# EUROPÄISCHE PATENTSCHRIFT

(11) **EP 0 517 024 B1**
(45) Veröffentlichungstag und Bekanntmachung des Hinweises auf die Patenterteilung: **13.08.1997**
(21) Anmeldenummer: 92108381.2
(22) Anmeldetag: 18.05.1992
(51) Int. Cl.: C12N 15/13, C07K 14/00

(54) **Tetravalente bispezifische Rezeptoren, ihre Herstellung und Verwendung**
Tetravalent bispecific receptors, their preparation and use
Récepteurs tétravalents bispécifiques, leur préparation et utilisation

(30) Priorität: 03.06.1991 DE 4118120
(43) Veröffentlichungstag der Anmeldung: 09.12.1992
(73) Patentinhaber: BEHRINGWERKE Aktiengesellschaft, 35001 Marburg (DE)
(72) Erfinder: Bosslet, Klaus, W-3550 Marburg (DE); Seemann, Gerhard, W-3550 Marburg-Elnhausen (DE)
(74) Vertreter: Fischer, Hans-Jürgen, Dr.

(56) Entgegenhaltungen:
- EP-A- 0 404 097
- EP-A- 0 419 387
- WO-A-91/03493
- CANCER RESEARCH, Bd. 50, Nr. 11, 1. Juni 1990, PHILADELPHIA PA, VS, Seiten 3445-3452; J. LE DOUSSAL ET AL.: 'Targeting of Indium 111-labeled bivalent hapten to human melanoma mediated by bispecific monoclonal antibody conjugates: Imaging of tumors hosted in nude mice'.
- BRITISH JOURNAL OF CANCER, Bd. 63, Nr. 5, Mai 1991, LONDON, GB, Seiten 681-686; K. BOSSLET ET AL.: 'Generation of bispecific monoclonal antibodies for two phase radioimmunotherapy.'
- JOURNAL OF NUCLEAR MEDICINE, Bd. 32, Nr. 5SUP, Mai 1991, NEW YORK, VS, Seiten 915-916; L. ANDERSON ET AL.: 'Comparison of DOTA and DTPA analogs for bifunctional antibody delivery of Indium-111 and Yttrium-90.'

## Beschreibung

Die Erfindung betrifft tetravalente bispezifische Rezeptoren, die gentechnisch durch Fusion der DNA, die für die schwere Kette eines F(ab)'₂ Fragmentes eines Antikörper (I) kodiert mit (a) DNA, welche für die schwere Kette eines F(ab')₂ Moleküls eines zweiten Antikörpers (II) kodiert, bei dem die C_{H}1 Domäne durch eine C_{H}3 Domäne ersetzt ist (Formel I), oder mit (b) der DNA, die für ein "single chain" F_{V}-Fragment eines Antikörpers (II) kodiert (Formel II), mittels geeigneter Linker hergestellt werden. Die Expression dieser Fusionsgene in Säugerzellen zusammen mit den Genen für die entsprechenden leichten Ketten, welche im Falle des Konstruktes (a) zum einen aus einem V_{L} Exon der Spezifität I und einem C_{K} Exon und zum anderen aus einem V_{L} Exon der Spezifität II und einem C_{H}3 Exon und im Falle des Konstruktes (b) nur aus einem V_{L} Exon der Spezifität I und einem C_{K} Exon besteht, ergibt tetravalente bispezifische Rezeptoren. Die C_{H}1 Domänen sind dabei mit den V_{H}2 Domänen über 1 bis 10 Hinge-Regionen (H) und einen geeigneten Peptidlinker L verbunden. Vorzugsweise werden die in der Europäischen Patentanmeldung EP-A2-0404 097 beschriebenen Antikörper-Spezifitäten eingesetzt. Dies sind u.a. zum einen Spezifitäten, die gegen ein auf der Zellmembran oder im Interstitium befindliches Epitop eines tumorassoziierten Antigens gerichtet sind. Zum anderen sind dies Spezifitäten, die gegen hochmolekulare oder niedermolekulare Liganden gerichtet sind, die ihrerseits ein gegen Tumoren wirksames Agens binden oder aber direkt dieses Agens binden.

In der EP-A2-0404 097 werden bispezifische und oligospezifische, mono- und oligovalente Rezeptoren beschrieben, die gentechnisch durch Fusion von für F(ab) Fragmente von Antikörper zweier oder mehrerer verschiedener Spezifitäten kodierende DNA mittels geeigneter Linker hergestellt werden. Vorzugsweise ist eine Spezifität dabei entweder gegen ein auf der Zellmembran oder im Interstitium befindliches Epitop eines Tumorassoziierten Antigens (TAA) oder gegen ein Epitop im Tumorendothel (TE) gerichtet, während die weiteren Spezifitäten hochmolekulare oder niedermolekulare Liganden betreffen und z.B. mit den Komplexonen Äthylendiamintetraacetat bzw. Diäthylentriaminpentaacetat in Yttrium 90 komplexierter Form (EDTA-⁹⁰Y bzw. DTPA-⁹⁰Y) reagieren. In einer besonders bevorzugten Ausführungsform erfolgt die Bindung mit den Komplexonen am Komplexon-Rezeptor-Arm über fos-jun Interaktion (oder auch Avidin-Biotin Interaktion). Weitere bevorzugte Spezifitäten haben katalytische Eigenschaften.

Bispezifische Antikörper wurden bisher nach folgenden Methoden hergestellt
- chemische Kopplung von Antikörpern verschiedener Spezifität über heterobifunktionelle Linker (H. Paulus, Behring Inst. Mitt. 78, (1985), 118-132)
- Fusion von bereits vorhandenen Hybriden, die verschiedene monoklonale Antikörper (MAK) ausscheiden, und Isolation des bispezifisch-monovalenten Anteiles (U.S. Staerz und M.J. Bevan, Proc. Natl. Acad. Sci. USA 83, (1986) 1453-1457)
- Transfektion der leichten und schweren Kettengene zweier verschiedener MAK (4Gene) in murine Myelomzellen oder andere eukaryotische Expressionssysteme und Isolation des bispezifisch-monovalenten Anteiles (U. Zimmermann, Rev. Physio. Biochem. Pharmacol. 105 (1986), 176-260; J. van Dijk et al., Int. J. Cancer 43, (1989), 944-949).

Solche bispezifische Antikörper werden zur Therapie und Diagnostik von malignen Tumoren eingesetzt. Das Prinzip des Verfahrens besteht darin, daß im ersten Schritt durch Injektion des bispezifischen Makromoleküls über längere Zeiträume und mit hohen Dosen eine Absättigung der Epitope, die von einer der beiden Spezifitäten auf den Zielzellen erkannt werden, erreicht wird. Im zweiten Schritt, der aus einer mehrtägigen Unterbrechung der Behandlung besteht, findet die Autoelimination des unspezifisch adsorbierten bispezifischen Antikörpers aus den Nicht-Zielgeweben statt.

Diese Autoelimination kann durch Injektion eines mit Zuckerresten, vorzugsweise Galactose gekoppelten antiidiotypischen Antikörpers, der gegen den Anti-Tumor-Arm des bispezifischen Rezeptors gerichtet ist, beschleunigt werden.

Der dritte Schritt des Verfahrens besteht in der i.v. Injektion eines radiomarkierten, hydrophilen, sich nicht in Zellen anreichernden niedermolekularen Liganden mit kurzer Verweilzeit im Organismus, der hohe Komplexkonstanten für beta- und gamma-Strahler wie ⁹⁰Y, ¹⁸⁶Re, ¹⁸⁸Re, ¹⁸⁹Re, ^{99m}Tc oder ¹¹¹In hat und an den die zweite Spezifität des bispezifischen Antikörpers mit hoher Affinität bindet. Durch diesen Schritt wird eine Anreicherung des radioaktiven Liganden, verbunden mit längerem Verbleib am Zielgewebe erreicht, was die selektive Zerstörung des Zielgewebes zur Folge hat bzw. eine Diagnostik beispielsweise von Metastasen ermöglicht.

Wir haben gefunden, daß tetravalente bispezifische Rezeptoren gemäß Formel I oder Formel II besonders gut gentechnisch herstellbar sind, da sie wesentlich effizienter exprimiert werden als andere Konstrukte. Hinzu kommt, daß die Avidität der ursprünglichen Antikörper zu den entsprechenden Antigenen bei den erfindungsgemäßen Konstrukten erhalten bleibt. Bevorzugt sind Konstrukte mit 1 bis 5, ganz bevorzugt 1 Hinge Region zwischen C_{H}1 und V_{H}2, da solche Konstrukte besonders effizient in BHK-Zellen exprimiert werden. Als Linker werden vorzugsweise die Sequenzen entsprechend der Peptidsequenz (Gly-Gly-Gly-Gly-Ser)ₓ mit x = 3 bis 5 oder GEAAPAAAPAAAAAGG eingesetzt. Im übrigen werden die in vorgenannter EP-A2-0404 097 beschriebenen oder bevorzugten Antikörper-V-Gen-Fragmente bzw. Spezifitäten auch hier vorzugsweise eingesetzt. 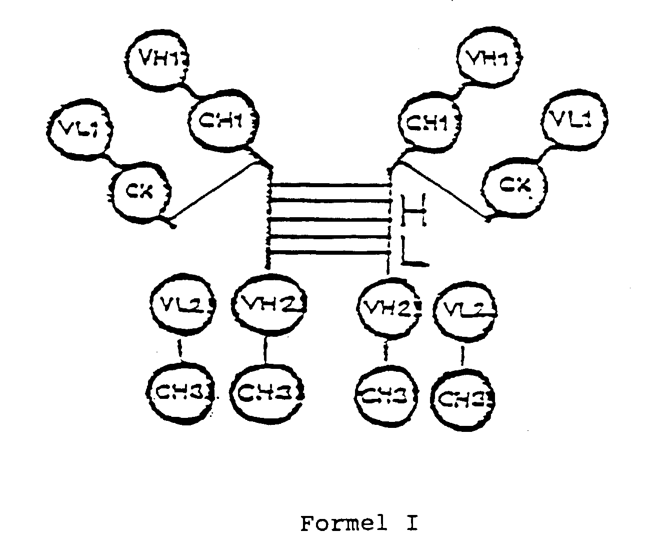 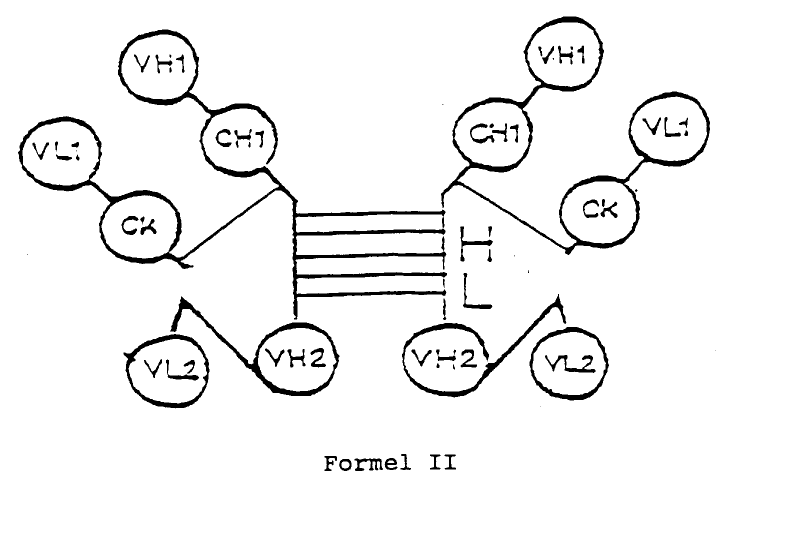
- H =: Hinge
- L =: Linker
- ― =: Peptidbindung
- ― =: Disulfidbrücke

Im folgenden wird beispielhaft die Konstruktion eines Fusionsgens beschrieben, welches für ein tetravalentes bispezifisches Rezeptormolekül kodiert. Wenn nicht anders vermerkt, sind die dabei verwendeten Techniken dem Buch "Molecular Cloning: A Laboratory Manual" entnommen (T. Maniatis et al., 1989). Weitere Einzelheiten sind in EP-A2-0404 097 beschrieben, auf die hier besonders Bezug genommen wird.

Die vorliegende Erfindung betrifft folglich bispezifische tetravalente Rezeptoren gemäß Formel I oder Formel II, Verfahren zu ihrer Herstellung und ihre Verwendung. Vorzugsweise ist eine Spezifität gegen animale oder humane tumorspezifische Antigene gerichtet

und die andere Spezifität besitzt katalytische oder enzymatische Aktivität oder ist gegen einen Komplexbildner gerichtet. In einer weiteren bevorzugten Ausführungsform stammt eine Spezifität von den monoklonalen Antikörpern mit den variablen Regionen gemäß Tab. 2, 3, 4 oder 5 aus EP-A2-0404 097 mit folgenden Sequenzen:

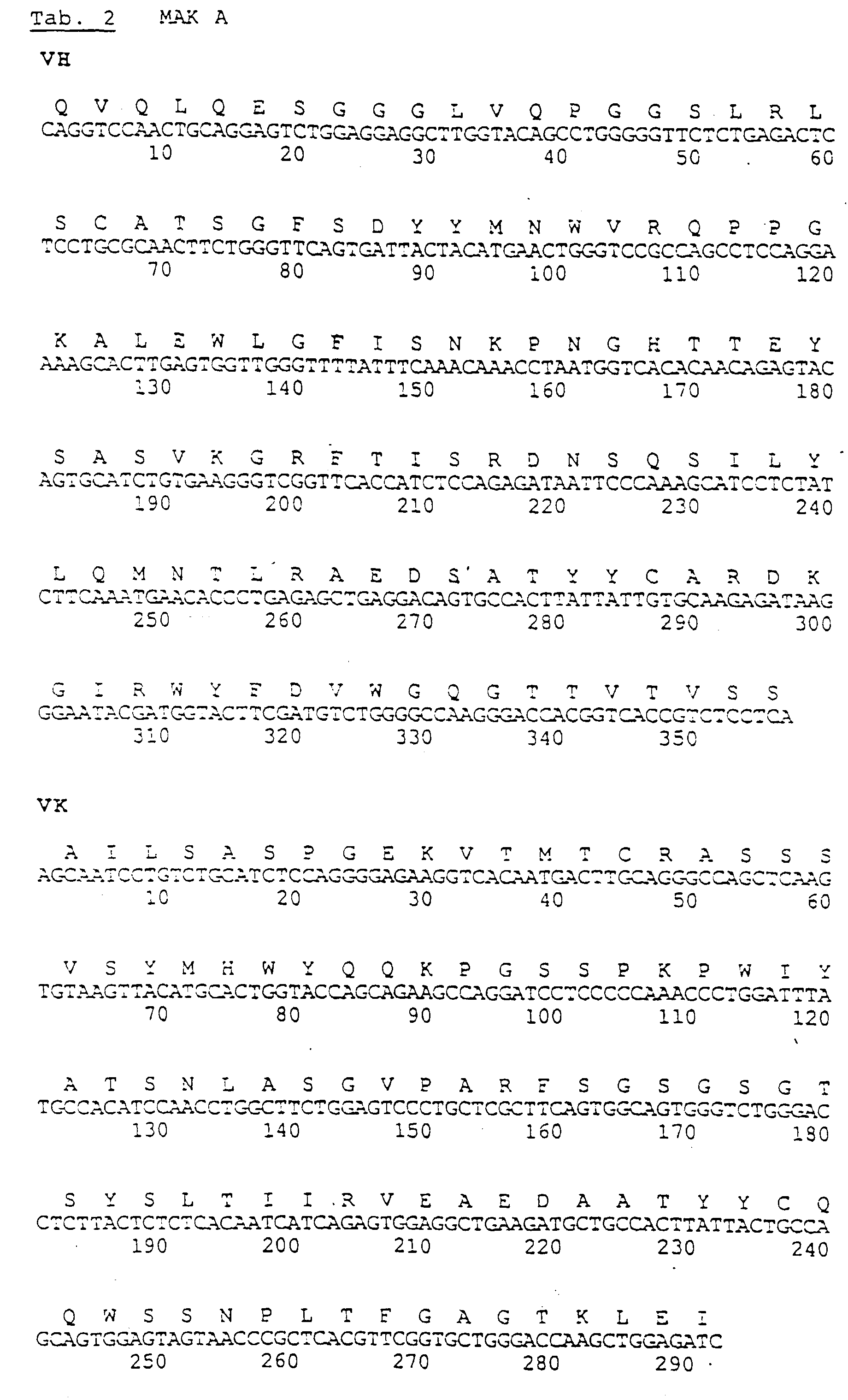

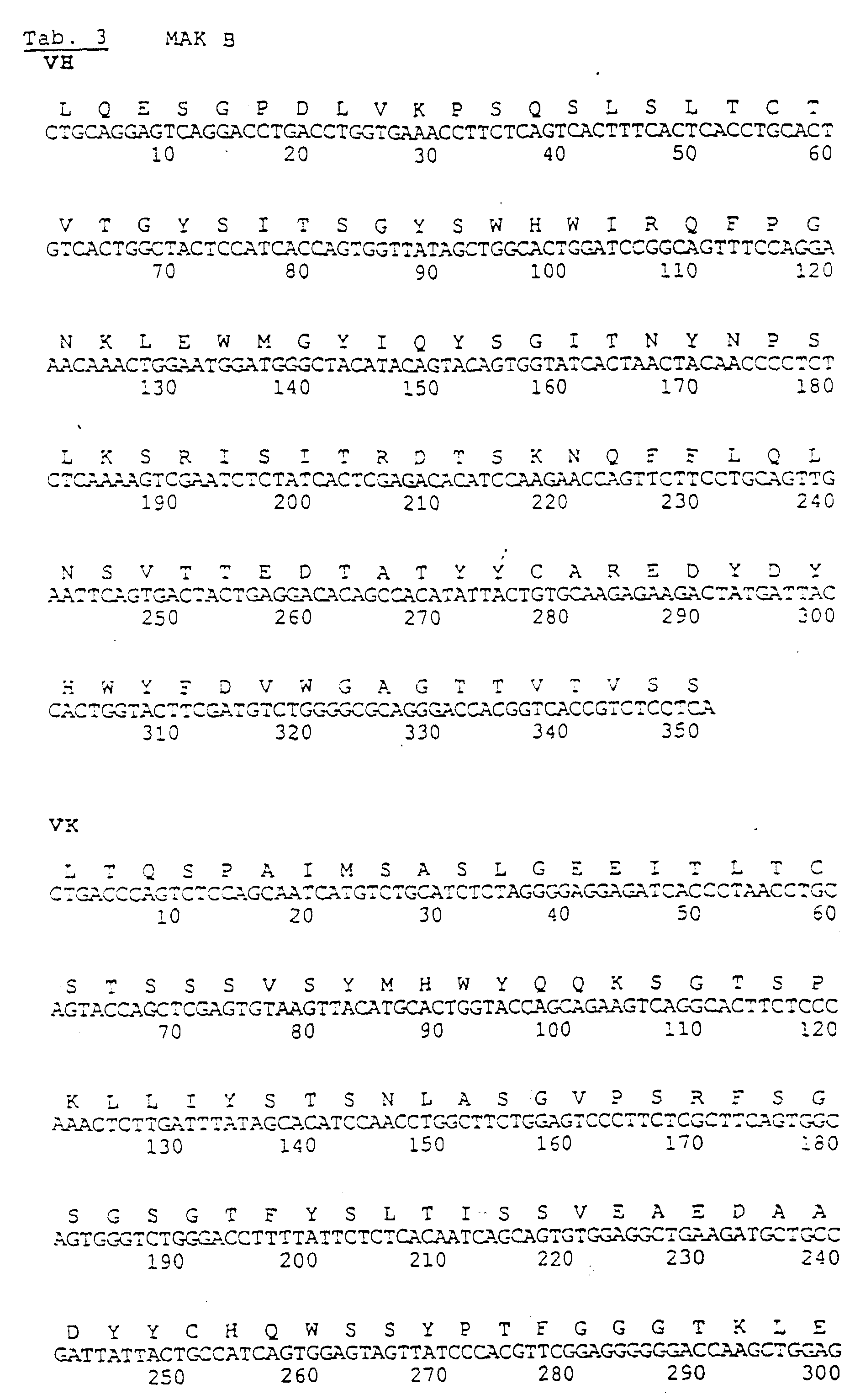

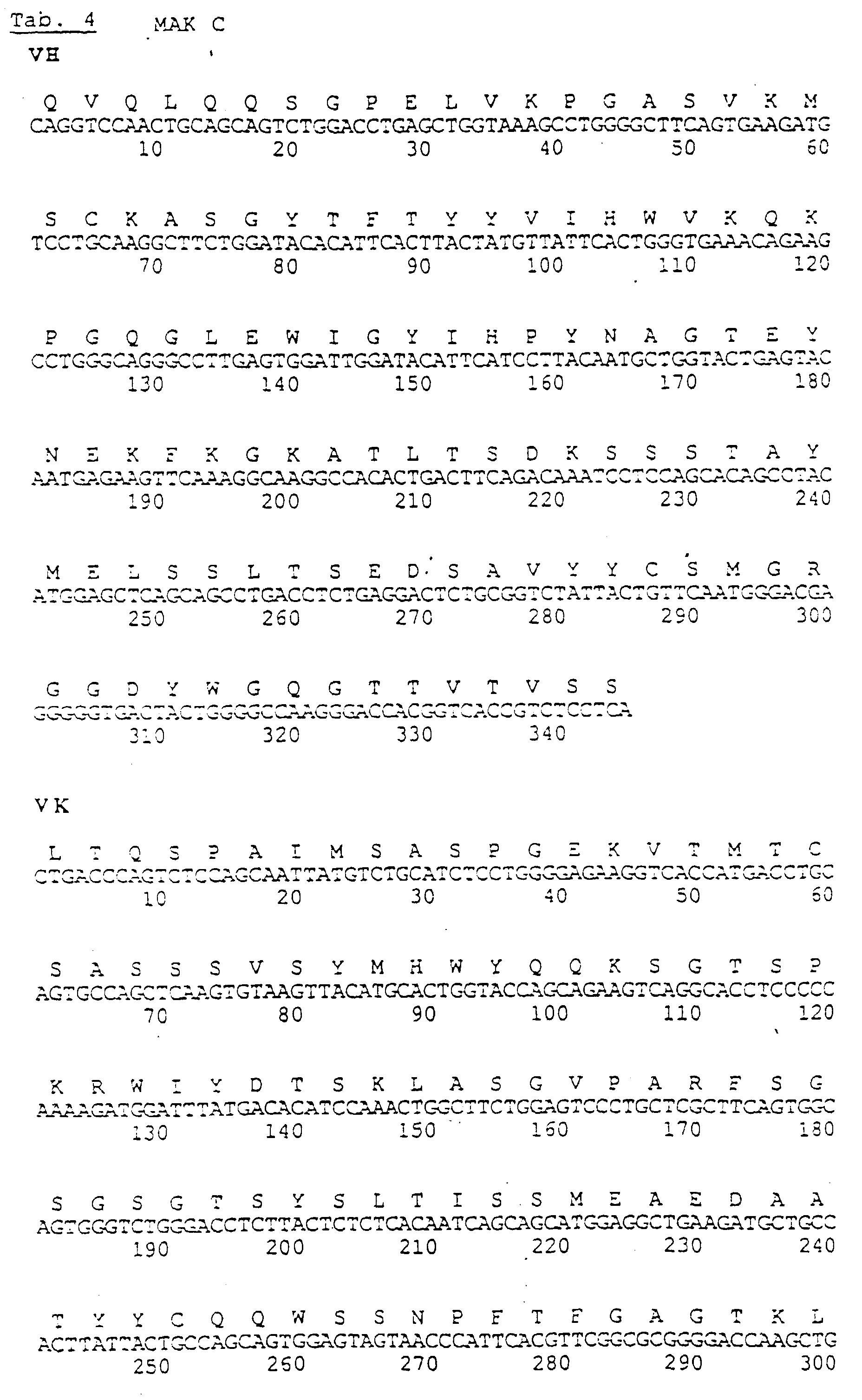

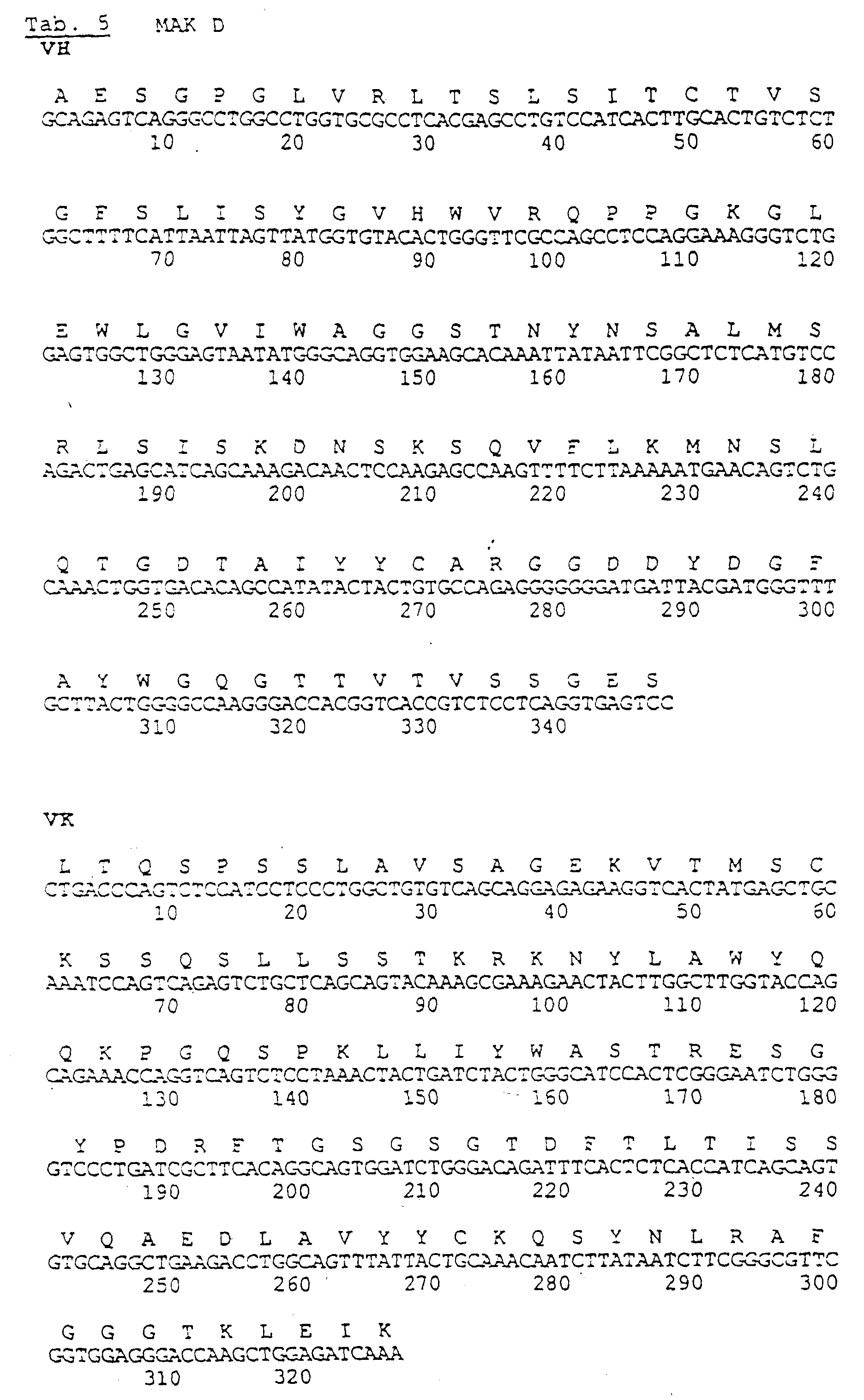

### Beispiel 1:

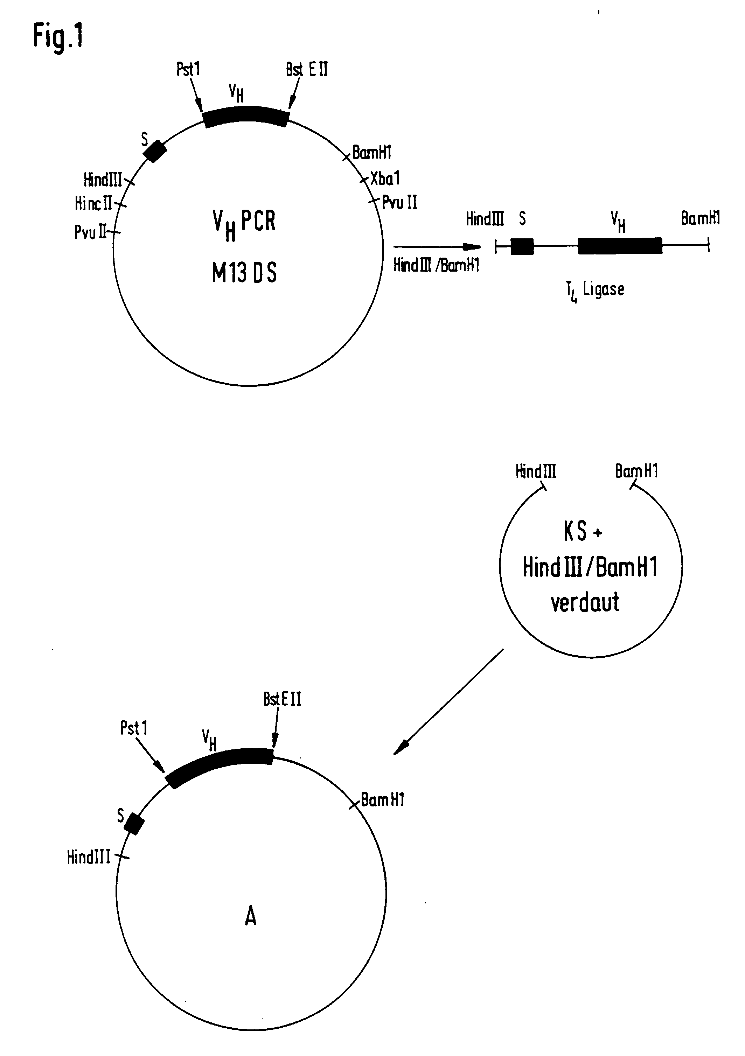

Ein Derivat eines M13 Phagen (V_{H} PCR), welches den 5' Teil eines schwere Ketten Gens bestehend aus Promoterregion, Signalexon, Intron 1, V_{H} Exon und Intron 2 enthält (R. Orlandi et al., Proc. Natl. Acad. Sci. USA, Vol. 86, 3833-3837, 1988) wurde mit den Restriktionsendonukleasen HindIII und BamHI gespalten, das Insert isoliert und in einen HindIII/BamHI gespaltenen KS+ Phasmid (pBluescript KS^{R}+, Stratagene LaJolla, CA, USA) kloniert. Durch Restriktionsanalyse und Nukleinsäuresequenzanalyse wurde der Phasmidklon (A) identifiziert, der das aus dem V_{H} PCR ausgeschnittene Insert enthält (Fig. 1).

### Beispiel 2:

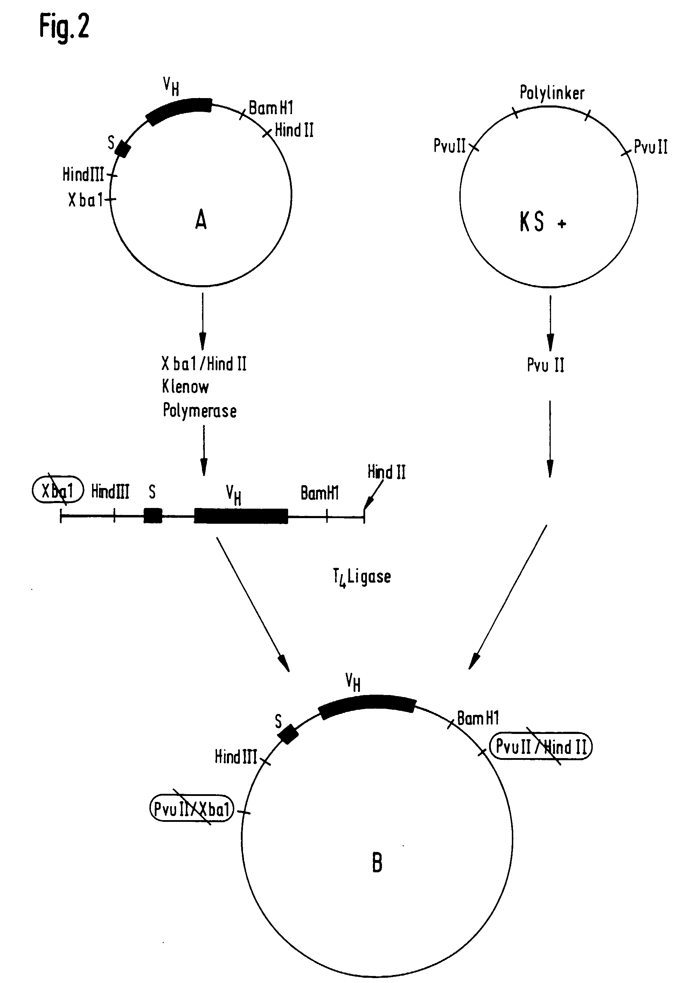

Der Phasmidklon (A) wurde mit XbaI und HindII gespalten, die XbaI Schnittstellen mit Klenow Polymerase und dNTPs aufgefüllt und das DNA Fragment mit dem V Gen Exon isoliert. Das isolierte DNA Fragment wurde dann in einen KS+ Phasmid kloniert, aus dem durch einen PvuII Verdau der Polylinker und dem Polylinker benachbarte Bereiche entfernt worden waren. Es wurde der Klon (B) identifiziert, der das V_{H} Insert und die zwischen den XbaI und BamH1 bzw. HindII und HindIII Schnittstellen gelegenen Bereiche des KS+ Polylinkers enthält (Fig. 2). Der Phasmid B wurde verwendet, um die V_{H} Gene der Antikörper I und II (V_{H}1 und V_{H}2) nach Amplifikation aus der cDNA der Hybridzellen zu klonieren. Die Amplifikation der V_{H} Gene erfolgte nach der von R. Orlandi et al. (1989, a.a.o.) beschriebenen Methode. Der Phasmidklon B mit dem V_{H}1 Gen wird als B1 und der Phasmidklon B mit dem V_{H}2 Gen als B2 bezeichnet (R.M. Hudziak et al., Cell., Vol. 31, 137-146, 1982; F. Lee et al., Nature, Vol. 294, 228, 1981).

### Beispiel 3:

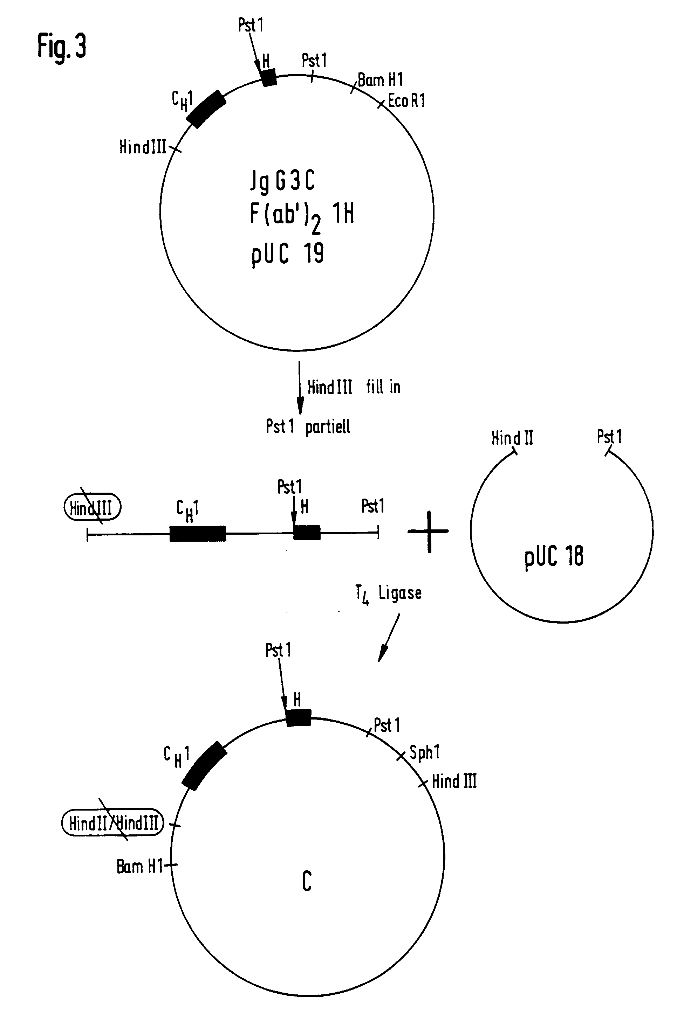

Ein pUC 19 Plasmid, der das C_{H}1 Exon und das erste Hinge Exon eines humanen IgG₃ Gens enthält (EP A2-0404097; Fig. 3 ibidem: IgG₃ (F(ab')₂ 1H)), wurde mit der Restriktionsendonuklease HindIII gespalten und die Schnittstellen mit Klenow DNA Polymerase aufgefüllt. Dann wurde mit PstI partiell gespalten, das DNA Fragment mit dem C_{H}1 Exon und dem H₁ Exon isoliert und in einen mit PstI und HindII gespaltenen pUC 18 Plasmid kloniert. Es wurde der Klon (C) isoliert, der 5' des Insert eine BamH1 und 3' des Inserts eine HindIII Schnittstelle trägt (Fig. 3).

### Beispiel 4:

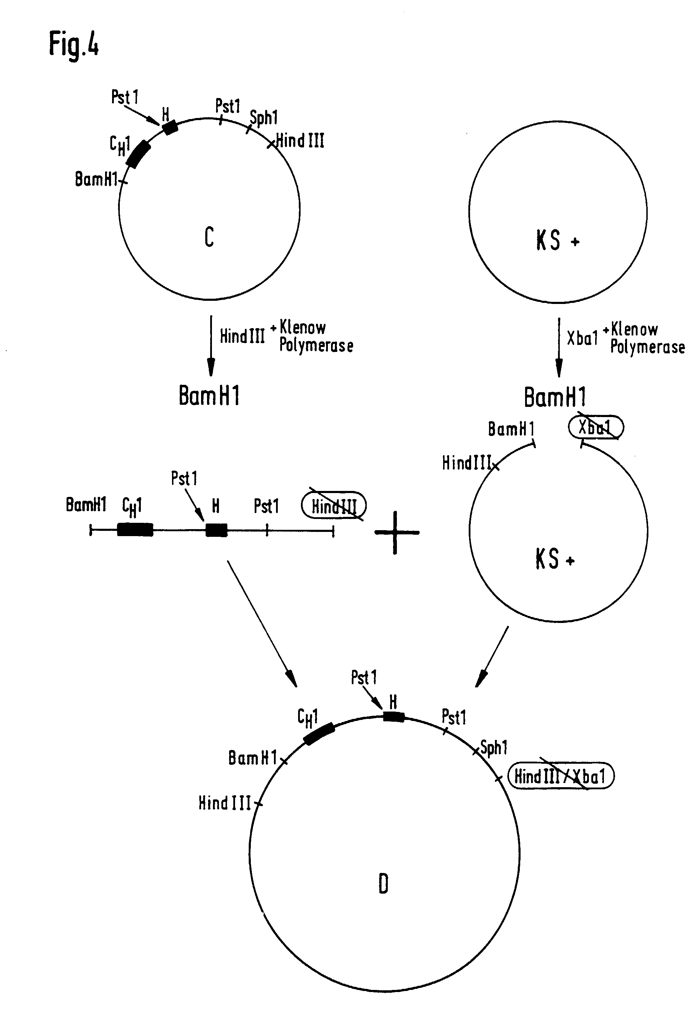

Der Plasmidklon C wurde mit HindIII gespalten, die Schnittstellen mit Klenow DNA Polymerase aufgefüllt. Dann mit BamH1 das Insert mit den C_{H}1 und H₁ Exons ausgeschnitten, isoliert und in einen KS+ Plasmid (pBluescript^{R} IIKS+, Stratagene, LaJolla, CA, USA) kloniert. Der KS+ Plasmid war mit XbaI gespalten, die XbaI Schnittstellen mit Klenow DNA Polymerase aufgefüllt und anschließend mit BamH1 nachgeschnitten worden. Es wurde der Phasmidklon (D) isoliert, der das Insert mit den C_{H}1 und Hinge 1 Exons in einer Orientierung enthält, bei der sich auf der 5' Seite des Inserts eine BamHI und eine HindIII Schnittstelle befinden (Fig. 4).

### Beispiel 5:

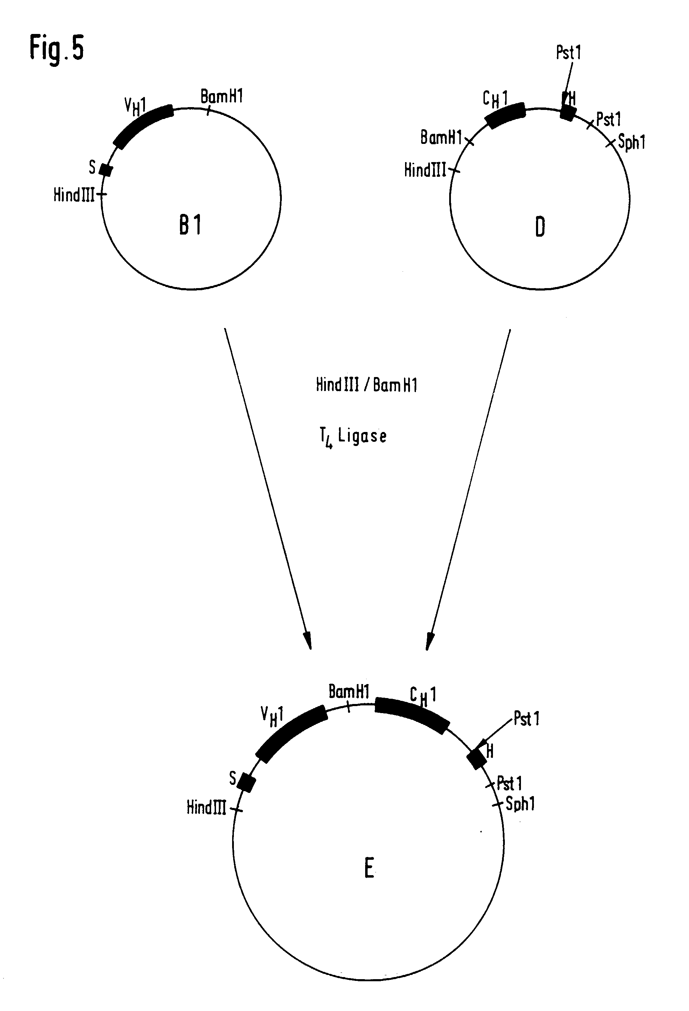

Der Phasmidklon (B1) wurde mit HindIII und BamH1 gespalten, das Insert mit dem V_{H} Gen des Antikörpers I isoliert und in den ebenfalls mit HindIII und BamHI gespaltenen Vektor (D) kloniert. Es wurde der Klon (E) isoliert, der das V_{H}1 Gen, das C_{H}1 und das Hinge 1 Exon enthält (Fig. 5).

### Beispiel 6:

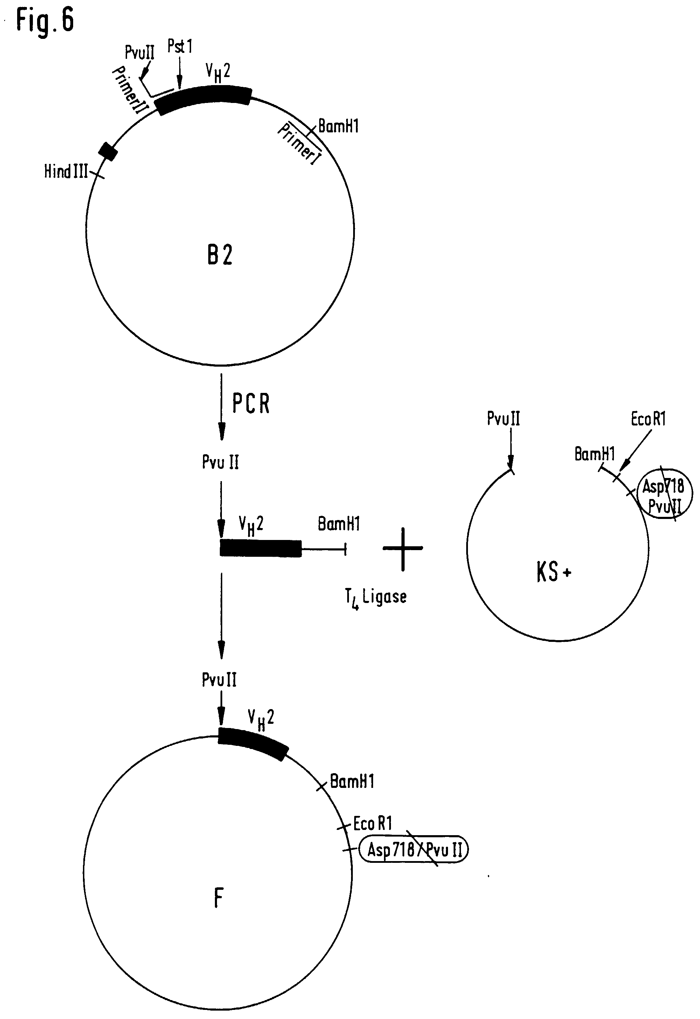

Aus dem Phasmidvektor B2 wurde mittels Polymerase Chain Reaction (PCR) unter Verwendung der Oligonukleotide V_{H}OligoI und V_{H}OligoII (Tab. 1) ein DNA Fragment amplifiziert (F), welches ein um einen Linker verlängertes V_{H} Gen enthält und an seinem 5' Ende eine gespaltene PvuII und an seinem 3' Ende eine BamH1 Schnittstelle trägt (Fig. 6). Dieses Fragment wurde mit BamH1 gespalten und in einen BamH1/PvuII gespaltenen KS+ Vektor kloniert, bei dem vorher eine der beiden internen PvuII Schnittstellen durch Asp718/PvuII Spaltung, Auffüllen der Schnittstellen und Religation zerstört worden war.

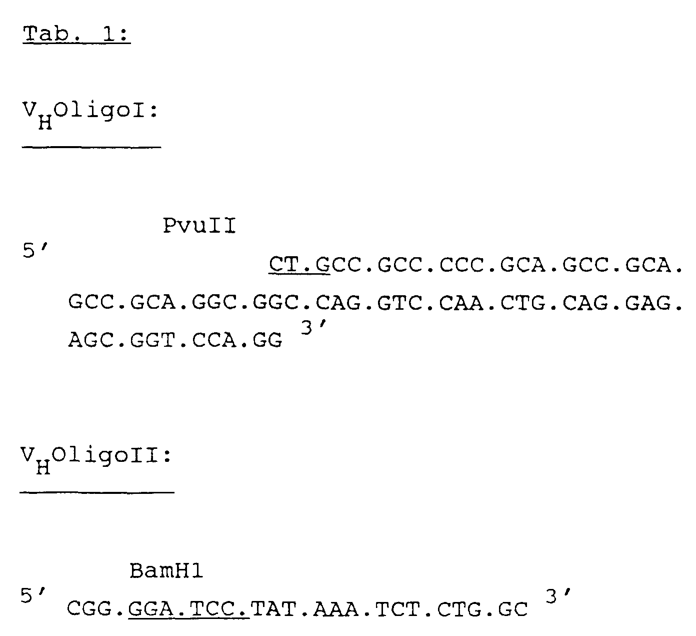

Es wurde der Phasmidklon (F) isoliert, der das amplifizierte Fragment enthält (Fig. 6).

### Beispiel 7:

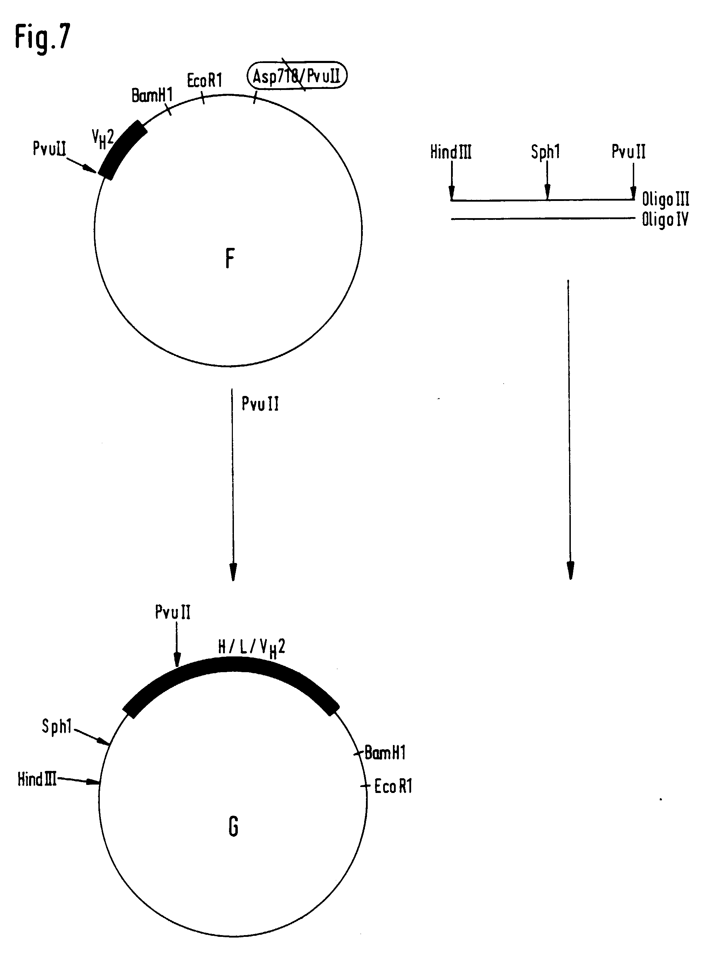

Die Oligonukleotide OligoIII und IV (Tab. 2) wurden miteinander hybridisiert und das entstehende DNA Fragment in den mit PvuII gespaltenen Phasmidklon (F) ligiert (Fig. 7). Es wurde der Phasmidklon (G) isoliert, der ein Fusionsexon aus einem Hinge Exon, einem Oligonukleotidlinker und dem V_{H}2 Gen enthält.

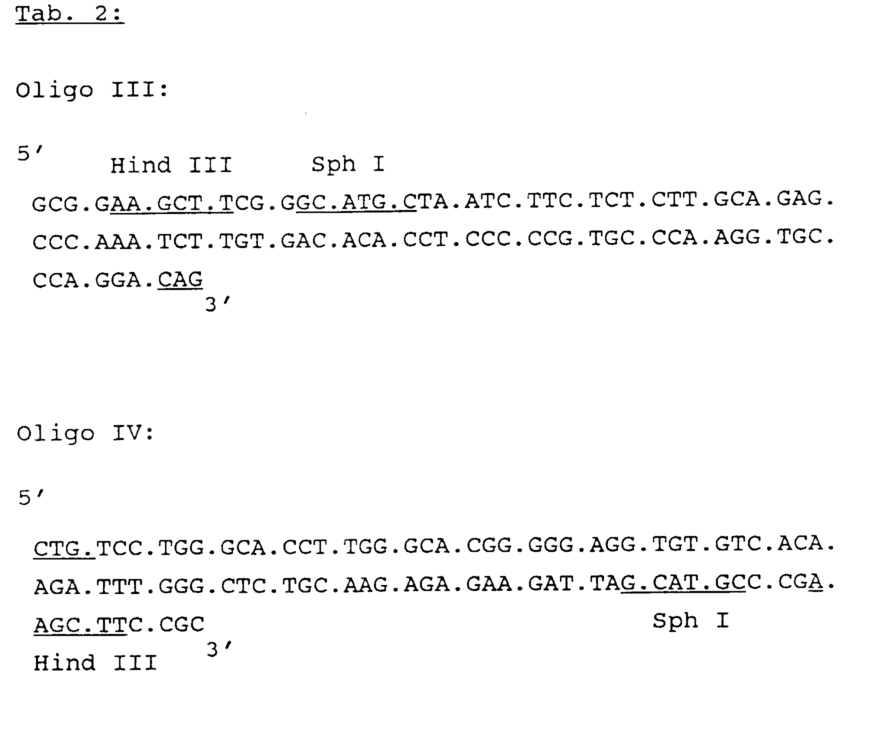

### Beispiel 8:

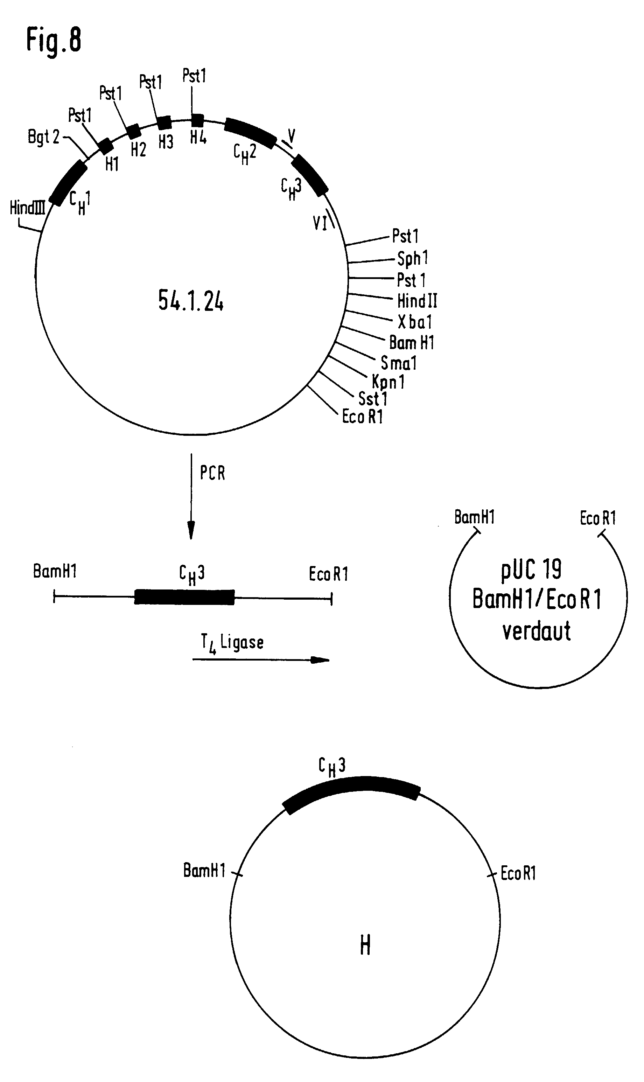

Aus dem Plasmidklon 54.1.24, der ein humanes IgG₃ C-Gen enthält (EP-A2-0404097, Fig. 2) wurde mit den Oligonukleotiden V und VI (Tab. 3) das C_{H}3 Exon und der 3' NT Bereich des IgG₃ Gens herausamplifiziert und in die BamH1 und EcoRI Schnittstellen eines pUC19 Plasmids kloniert (Fig. 8). Es wurde der Plasmidklon (H) isoliert, der das C_{H}3 Exon des IgG₃ Gens enthält.

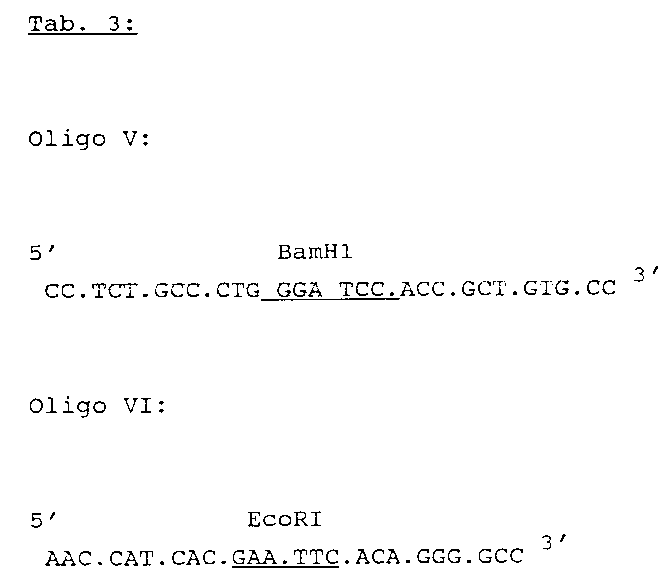

### Beispiel 9:

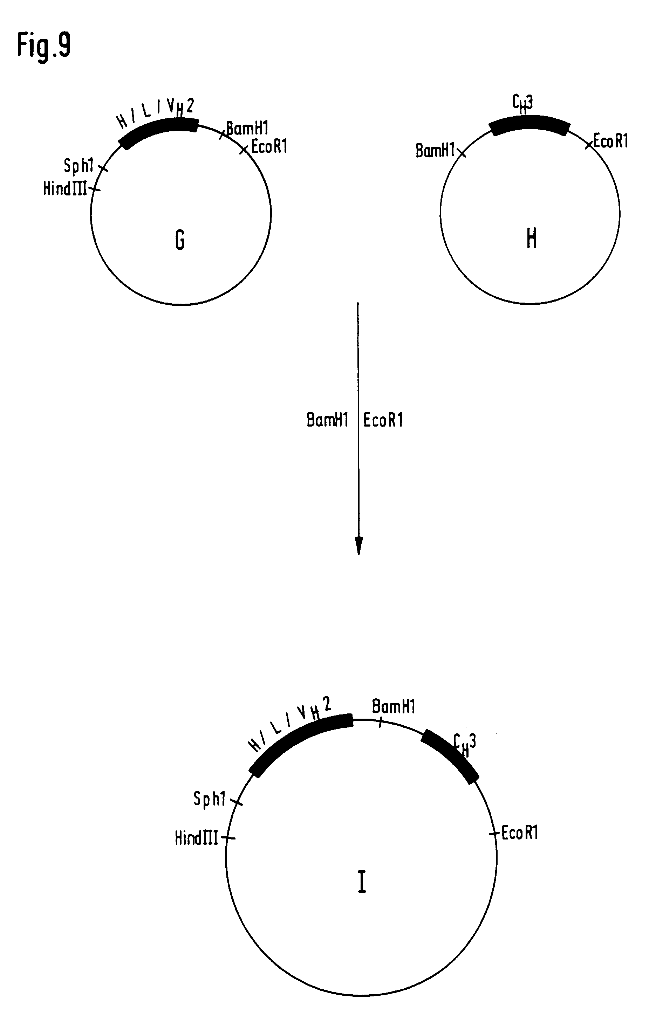

Der Plasmidklon (H) wurde mit BamHl und EcoRI gespalten und das DNA Fragment, welches das C_{H}3 Exon trägt, in den mit BamHl und EcoRI gespaltenen Phasmidklon (G) kloniert (Fig. 9). Es wurde der Phasmidklon (I) isoliert, der das Hinge/ Linker/V_{H}2 Fusionsexon und das C_{H}3 Exon enthält.

### Beispiel 10:

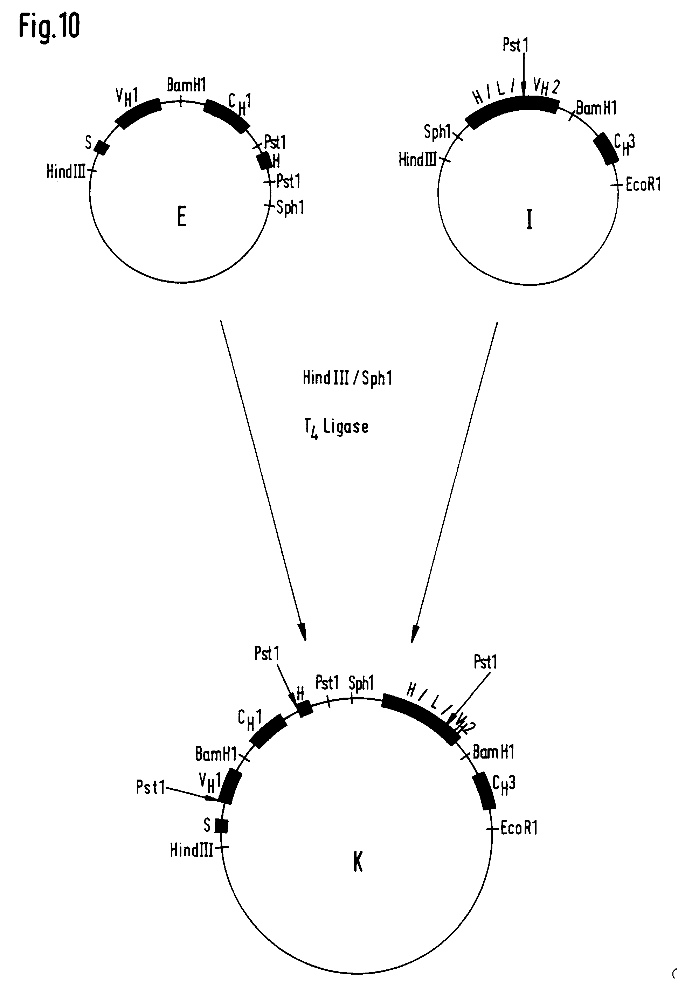

Die Phasmidklone (E) und (I) wurden mit HindIII und SphI gespalten. Das Insert des Klons (E) wurde in die HindIII und SphI Schnittstellen des Phasmidklons (I) kloniert. Es wurde der Phasmidklon (K) isoliert, der ein Ig schwere Ketten Fusionsgen enthält, welches aus Signalexon, V_{H}1 Exon, C_{H}1 Exon, Hingel Exon, Hinge/ Linker/V_{H}2 MAkII Fusionsexon und C_{H}3 Exon besteht (Fig. 10).

### Beispiel 11:

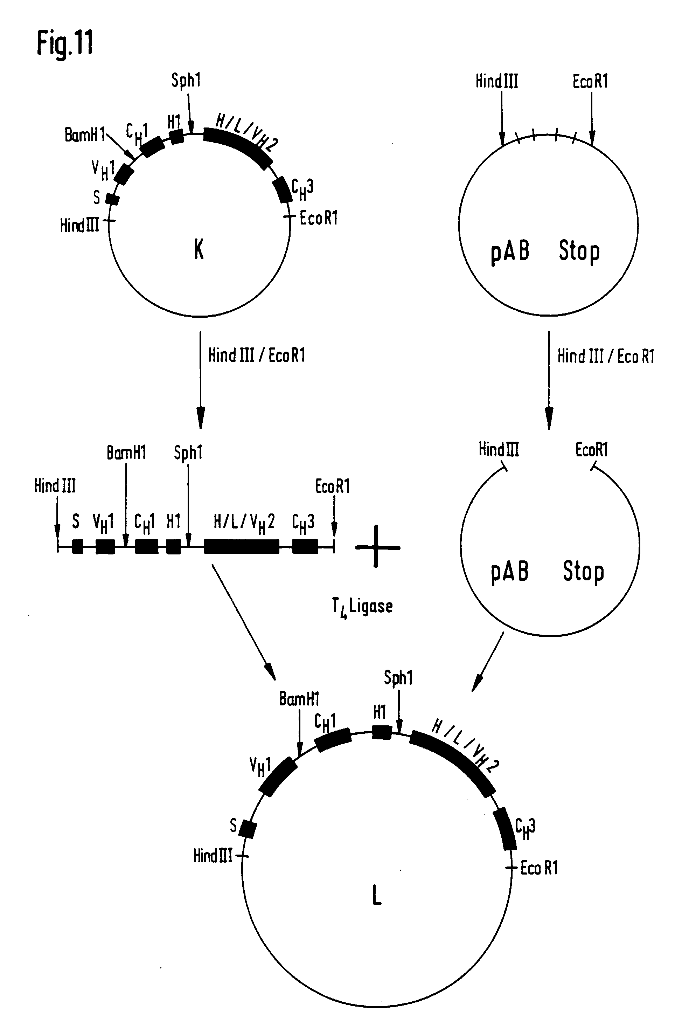

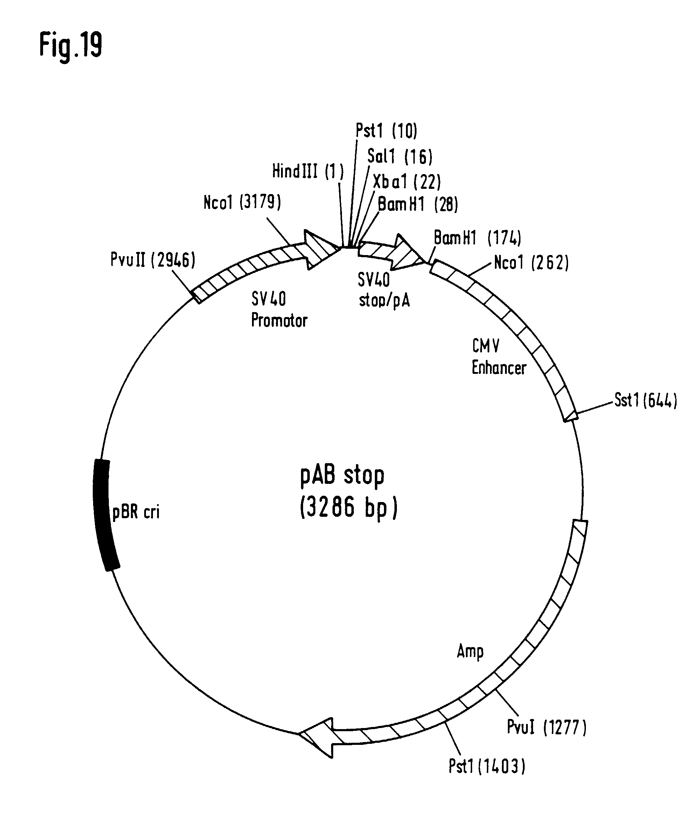

Aus dem Phasmidklon (K) wurde das HindIII-EcoRI Fragment mit dem Fusionsgen ausgeschnitten und in einen pAB Stop Expressionsvektor kloniert (Fig. 19, (pAB Stop ist ein Derivat des pAB 3 Vektors (G. Zettlmeißl et al., BIM, 82, 26-34, (1988), bei dem das AT III Gen durch einen Polylinker ersetzt wurde), dessen BamHI Fragment durch einen EcoRI Linker ersetzt worden war. Es wurde der Klon (L) isoliert, der das Fusionsgen enthält (Fig. 11). Der Klon (L) wurde aufgebaut und für Transfektionen in Säugerzellen verwendet.

### Beispiel 12:

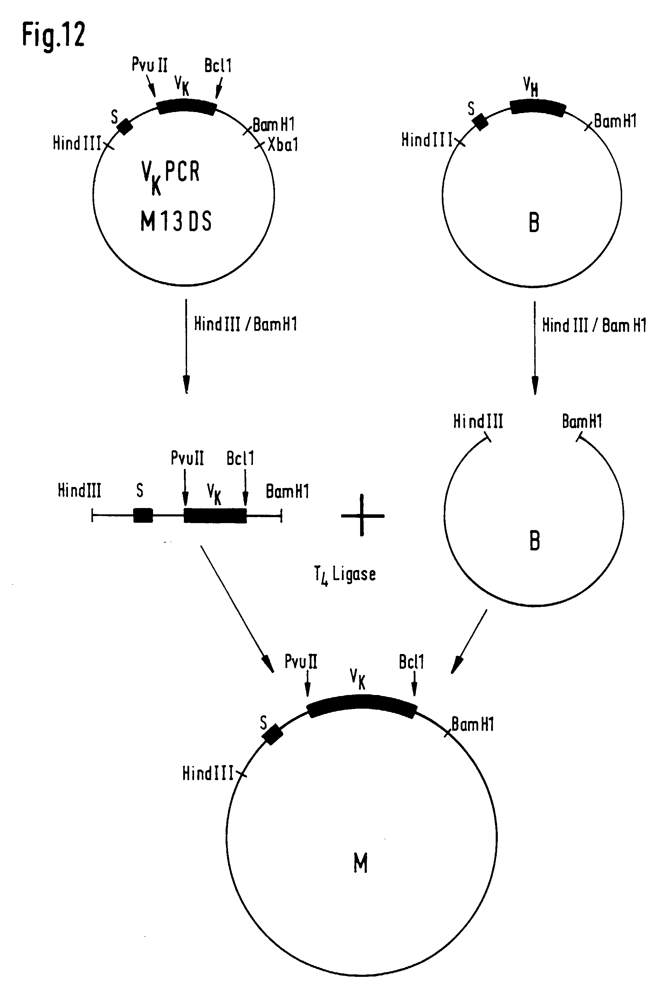

Konstruktion leichte Kette Gen:
Der Phasmidklon (B) wurde mit HindIII und BamH1 gespalten und das V_{H} Insert durch das aus dem Vektor V_{K}PCR (R. Orlandi et al. 1989, a.a.o.) isolierte V_{K} Insert ersetzt. Es wurde der Phasmidklon (M) isoliert, der ein Signalexon und ein V_{K} Exon trägt (Fig. 12). Der Phasmidklon wurde verwendet, um die amplifizierten V_{K} Gene der MAk I und II zu klonieren. Der Vektor M mit dem V_{K}1 Gen wurde als M1 und der Vektor M mit dem V_{K}2 Gen wurde als M2 bezeichnet.

### Beispiel 13:

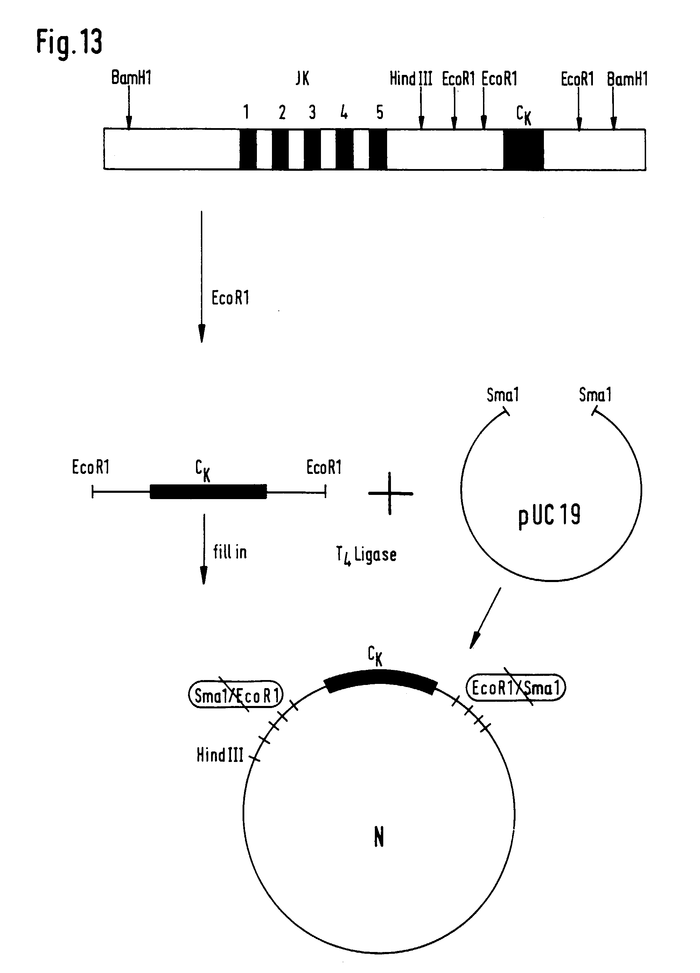

Das humane C_{K} Gen (Hieter et al., J. of Biol. Chem., 257: 1516-1522, 1982) wurde als EcoRI Fragment isoliert und in die SmaI Schnittstelle eines pUC 19 kloniert. Es wurde der Klon (N) isoliert, der das humane C_{K} Gen enthält (Fig. 13).

### Beispiel 14:

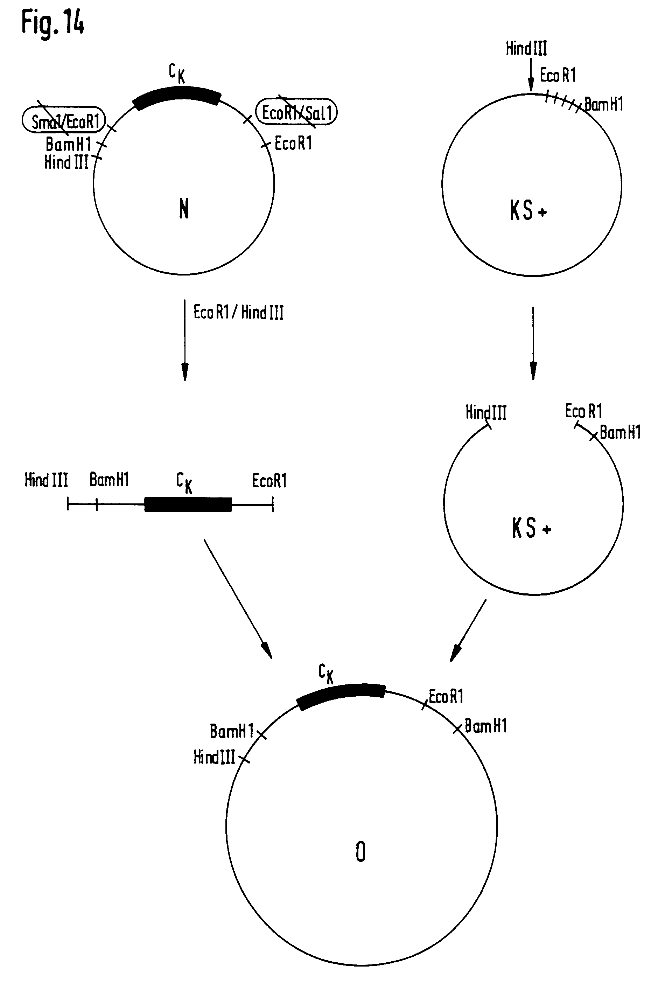

Der Klon (N) wurde mit EcoRI und HindIII gespalten, das C_{K} Insert isoliert und in einen EcoRI/HindIII gespaltenen KS+ Vektor kloniert. Es wurde der Phasmidklon (O) isoliert, der das C_{K} Insert enthält (Fig. 14).

### Beispiel 15:

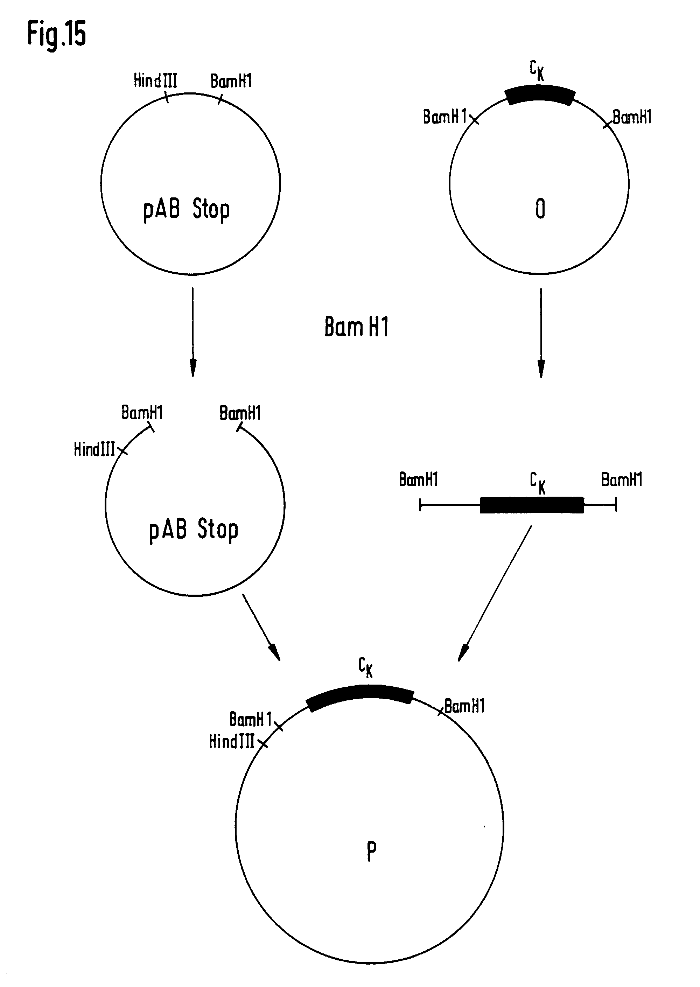

Der Klon (O) wurde mit BamHl gespalten, das C_{K} Insert isoliert und in einen BamH1 gespaltenen pAB Stop Vektor kloniert. Es wurde der Klon (P) isoliert, der das C_{K} Insert in einer Orientierung enthält, in der das 5' Ende des C_{K} Gens in der Nähe der HindIII Schnittstelle des pAB Stop Vektors liegt (Fig. 15).

### Beispiel 16:

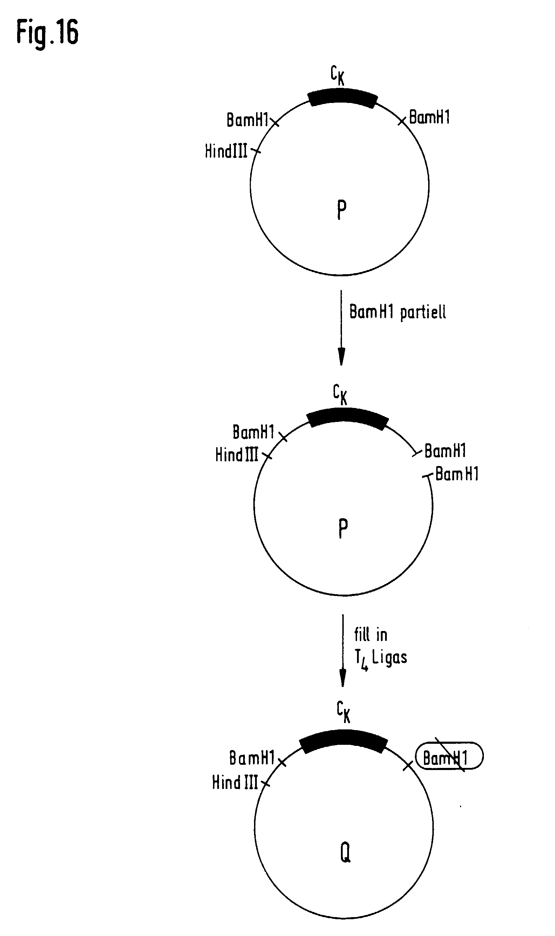

Der Klon (P) wurde partiell mit BamH1 gespalten, die Schnittstellen mit Klenow DNA Polymerase aufgefüllt und religiert. Es wurde der Klon (Q) identifiziert, bei dem die BamH1 Schnittstelle 3' des C_{K} Gens zerstört ist (Fig. 16).

### Beispiel 17:

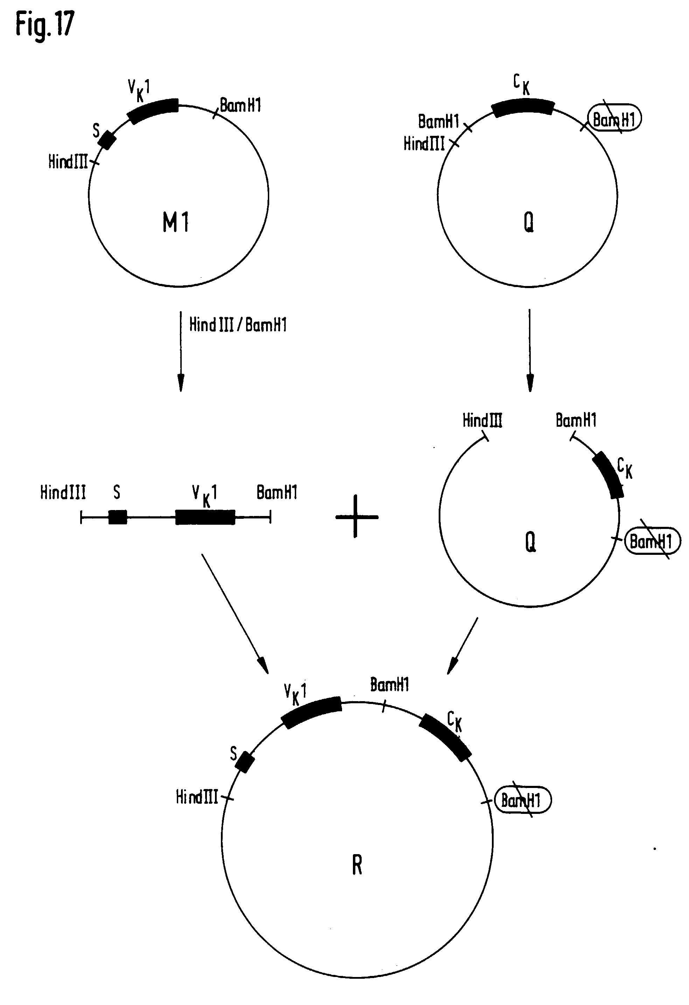

Der Phasmidklon (M1) mit dem V_{K}1 Gen wurde mit HindIII und BamH1 gespalten. Das Insert mit dem V_{K} Gen wurde isoliert und in die HindIII und BamH1 Schnittstelle des Expressionsvektors (Q) ligiert. Es wurde der Klon (R) identifiziert, der ein intaktes Kappa leichte Ketten Gen mit Spezifität des Antikörpers I enthält (Fig. 17).

### Beispiel 18:

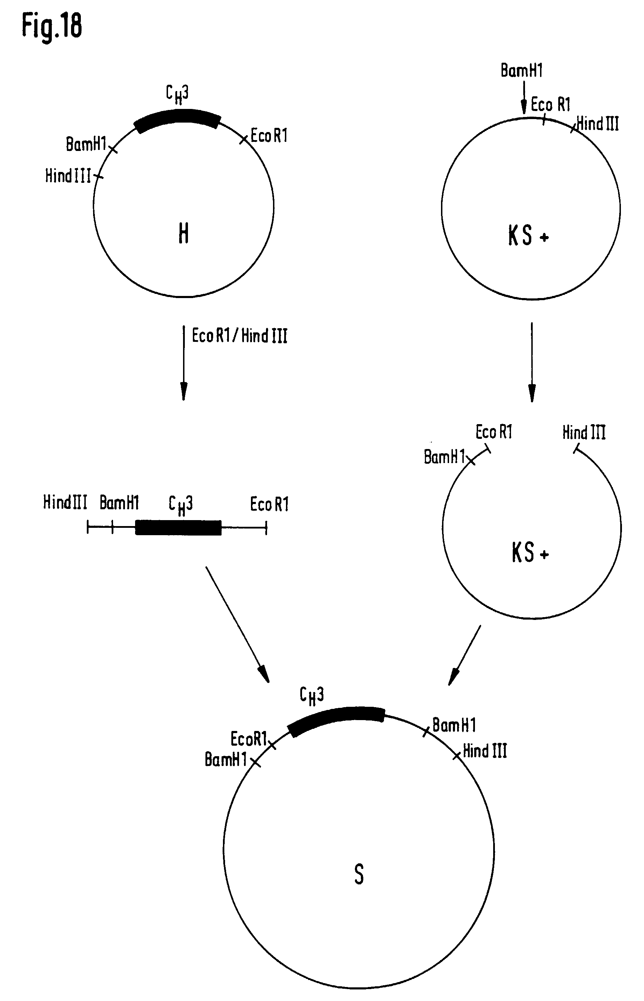

Der Plasmidklon (H) mit den C_{H}3 Exon des humanen IgG₃ Gens wurde mit EcoRI und HindIII gespalten, das C_{H}3 Insert isoliert und in einen EcoRI/HindIII gespaltenen KS+ Vektor kloniert. Es wurde der Phasmidklon (S) isoliert, der das C_{H}3 Insert enthält (Fig. 18).

### Beispiel 19:

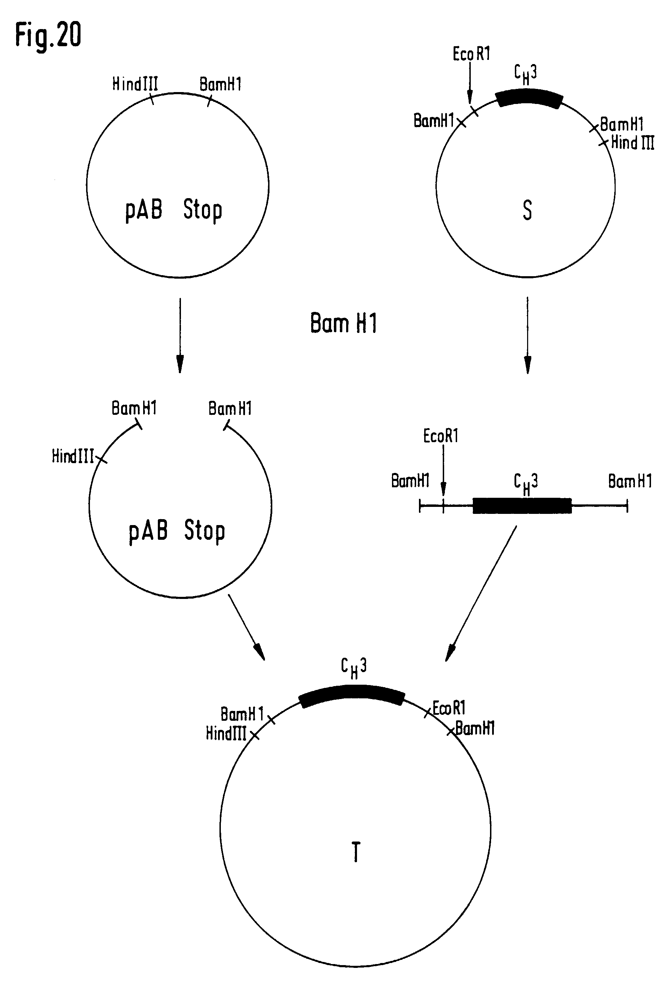

Der Klon (S) wurde mit BamH1 gespalten, das C_{H}3 Insert isoliert und in einen BamHl gespaltenen pAB Stop Vektor (Fig. 19) kloniert. Es wurde der Klon (T) isoliert, der das C_{H}3 Insert in einer Orientierung enthält, in der das 5' Ende des C_{H}3 Exons in der Nähe der HindIII Schnittstelle des pAB Stop Vektors liegt (Fig. 20).

### Beispiel 20:

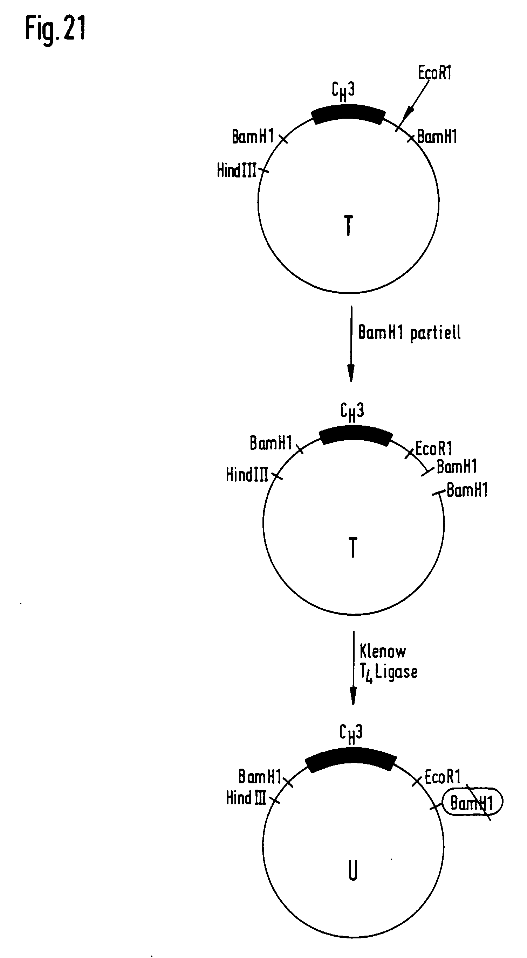

Der Klon T wurde partiell mit BamH1 gespalten, die Schnittstelle mit Klenow DNA Polymerase aufgefüllt und religiert. Es wurde der Klon (U) identifiziert, bei dem die BamH1 Schnittstelle 3' des C_{H}3 Gens zerstört ist (Fig. 21).

### Beispiel 21:

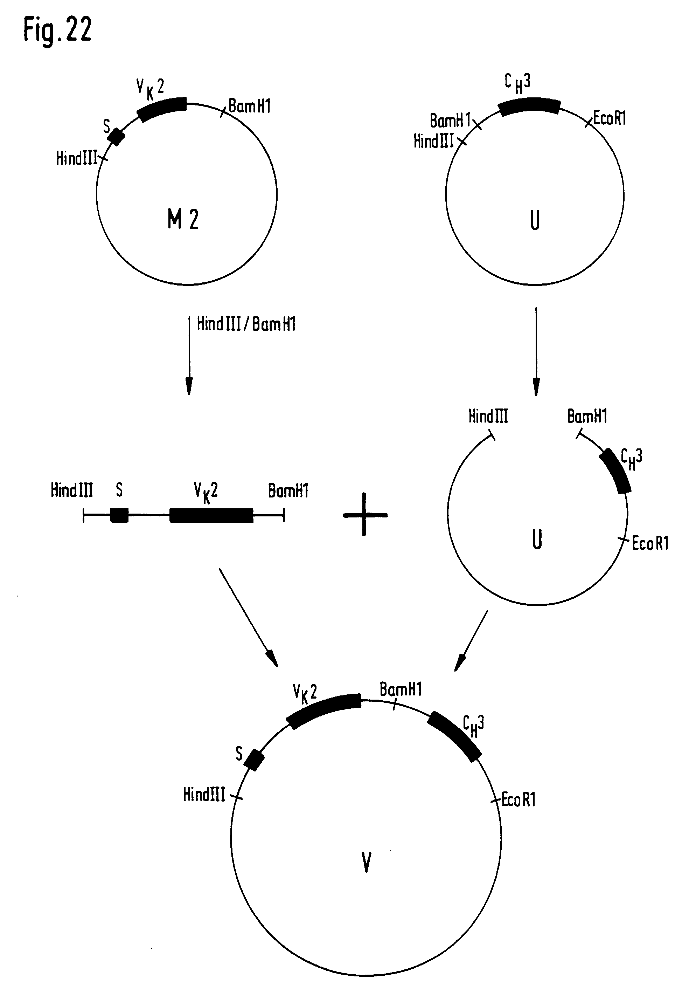

Der Phasmidklon (M2) mit dem V_{K}2 Gen wurde mit HindIII und BamH1 gespalten. Das Insert mit dem V_{K} Gen wurde isoliert und in die HindIII und BamHl Schnittstellen des Expressionsvektors (U) ligiert. Es wurde der Klon (V) identifiziert, der ein intaktes leichte Ketten Gen mit Spezifität des Antikörpers II und ein C_{H}3 Exon als konstante Region enthält (Fig. 22).

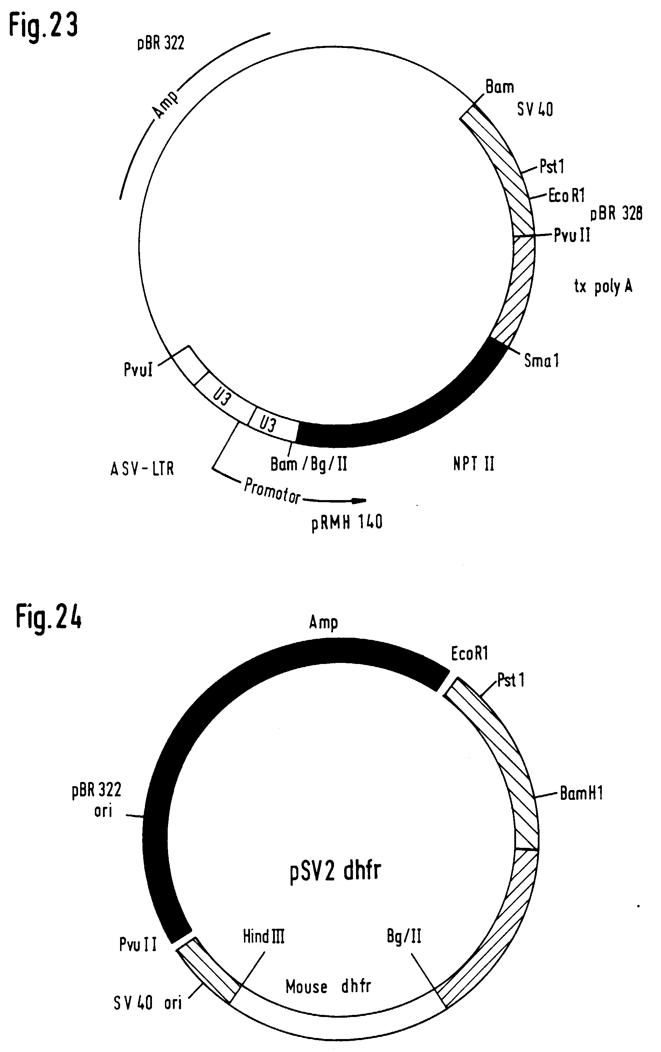

Die Expressionsplasmide L, R und V wurden zusammen mit geeigneter, Selektionsmarker-tragenden Phasmiden, wie z.B. pRMH140 (Fig. 23) (R.M. Hudziak et al., 1982, a.a.o.) oder pSV2dhfr (Fig. 24) (F. Lee et al., 1981, a.a.o.), in Säugerzellen kotransfiziert (30), durch Selektionsdruck Transfektomaklone selektioniert und durch Testung der Überstände mittels geeigneter Assays solche Transfektomaklone identifiziert, die bivalente tetraspezifische Rezeptormoleküle sezernieren.

## Patentansprüche (Patentansprüche für folgende(n) Vertragsstaat(en): AT, BE, CH, DE, DK, FR, GB, IT, LI, NL, LU, PT, SE)

1. Bispezifische, tetravalente Rezeptoren nach Formel I 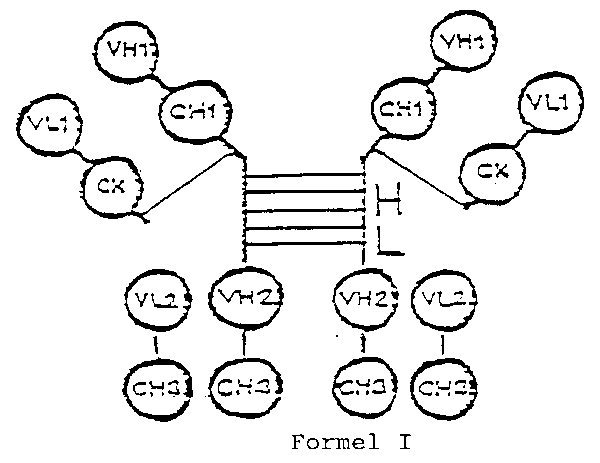 oder Formel II 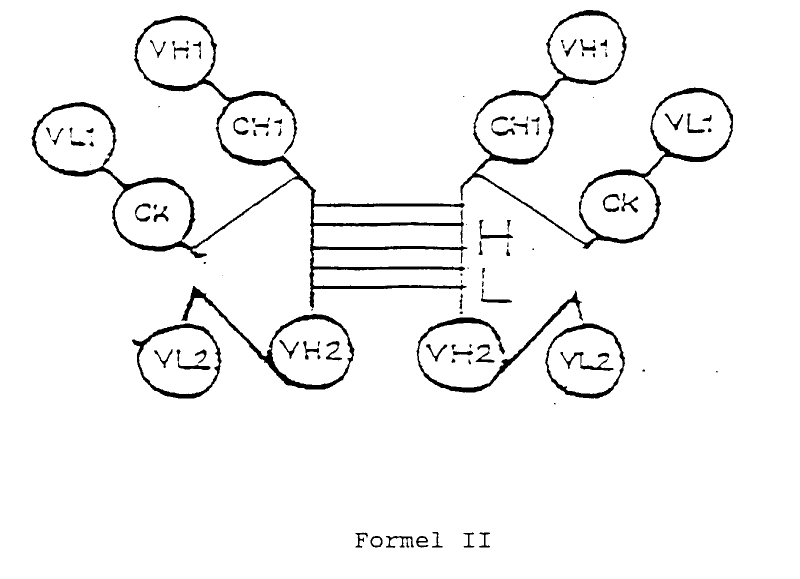 dadurch gekennzeichnet, daß nur die zusammengehörigen V_{L} und V_{H} -Paarungen gebildet werden und die C_{H}1 Domänen mit den V_{H}² Domänen über 1 bis 10 Hinge-Regionen H und einen geeigneten Peptidlinker L verbunden sind, wobei die Disulfidbrücken (dünne Striche) im Bereich der Hinge-Regionen die Dimerisierung der bivalenten Halbmoleküle bewirken.

2. Rezeptoren nach Anspruch 1, dadurch gekennzeichnet, daß eine Spezifität gegen animale oder humane tumorassoziierte Antigene gerichtet ist.

3. Rezeptoren nach Anspruch 1, dadurch gekennzeichnet, daß eine Spezifität katalytische oder enzymatische Aktivität besitzt.

4. Rezeptoren nach Anspruch 1, dadurch gekennzeichnet, daß eine Spezifität gegen animale oder humane tumorassoziierte Antigene gerichtet ist und die andere Spezifität katalytische oder enzymatische Aktivität besitzt.

5. Rezeptoren nach Anspruch 1, dadurch gekennzeichnet, daß eine Spezifität gegen animale oder humane tumorassoziierte Antigene gerichtet ist und eine andere Spezifität gegen ein Komplexon gerichtet ist.

6. Rezeptoren nach Anspruch 1, 2, 4 oder 5, dadurch gekennzeichnet, daß eine Spezifität von den monoklonalen Antikörpern mit den variablen Regionen gemäß Tab. 2, 3, 4 oder 5 mit folgenden Sequenzen stammt:

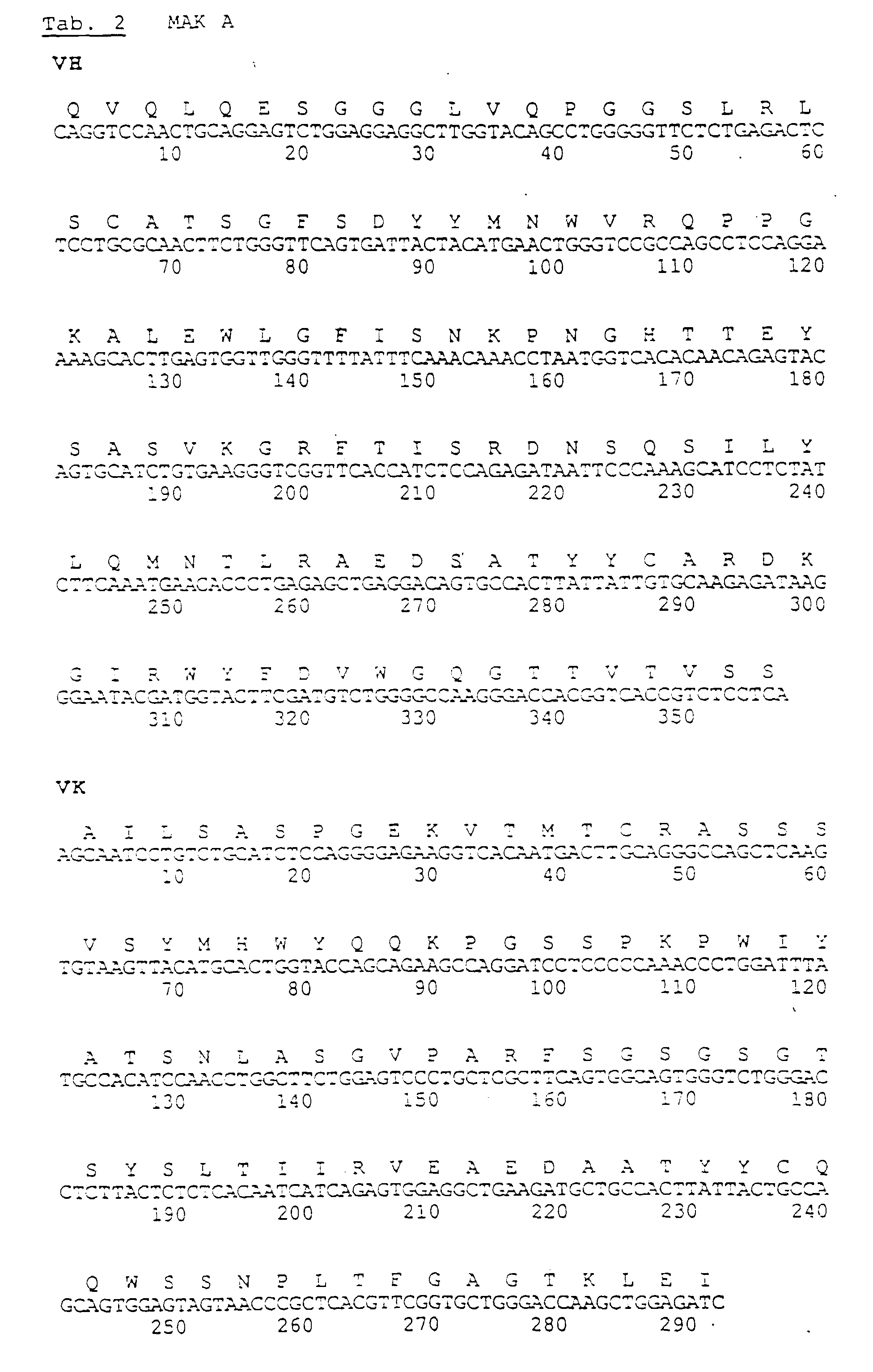

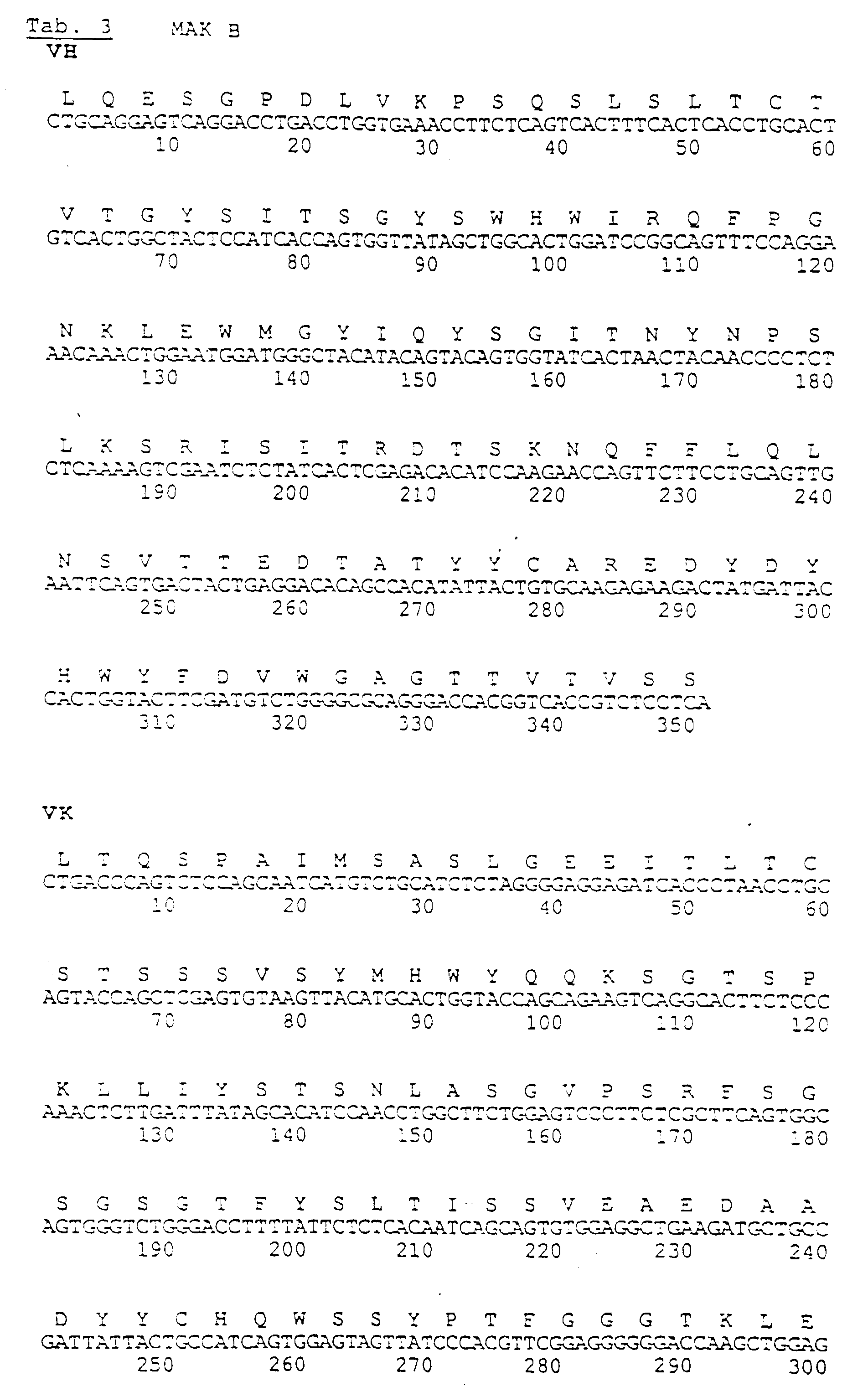

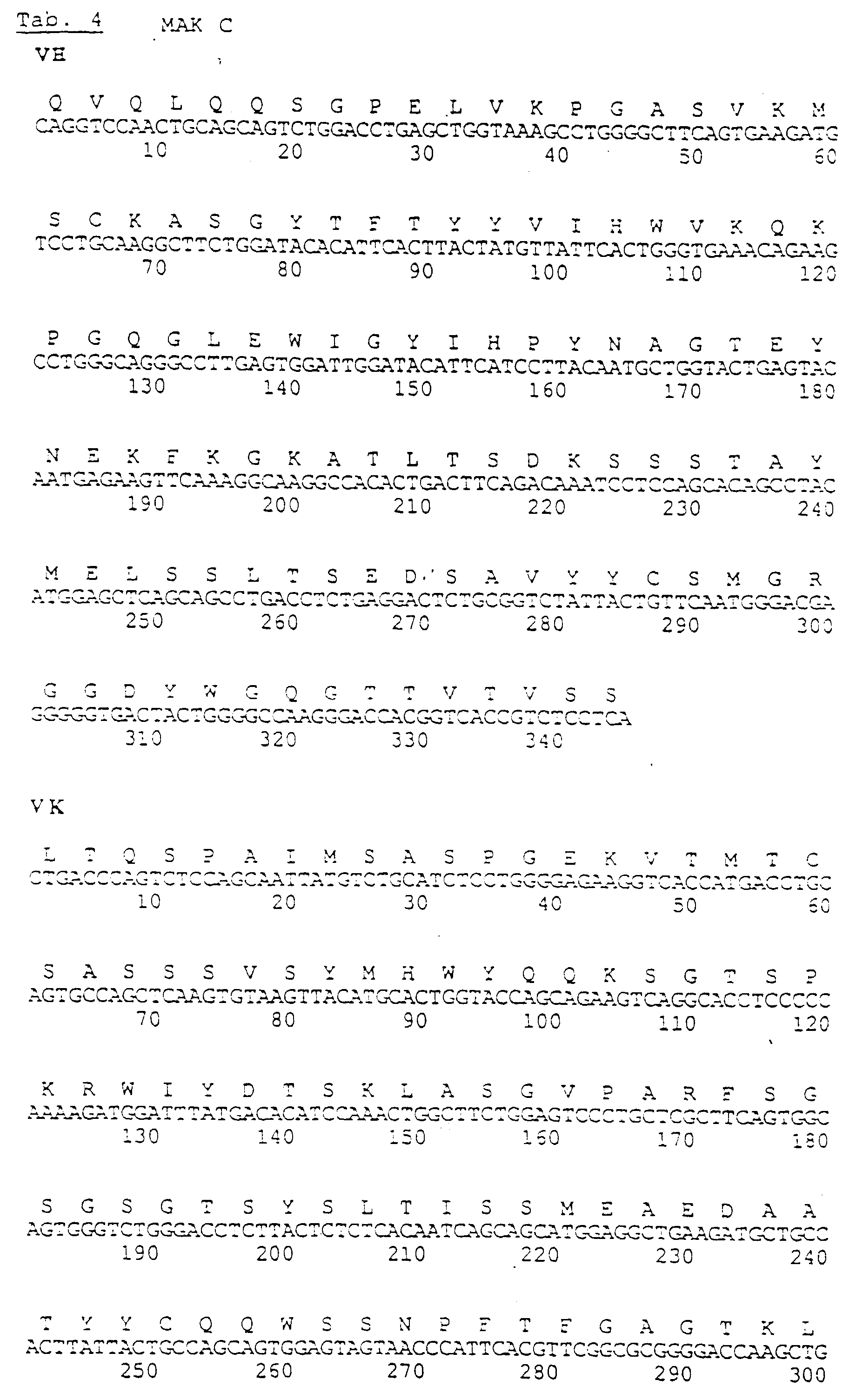

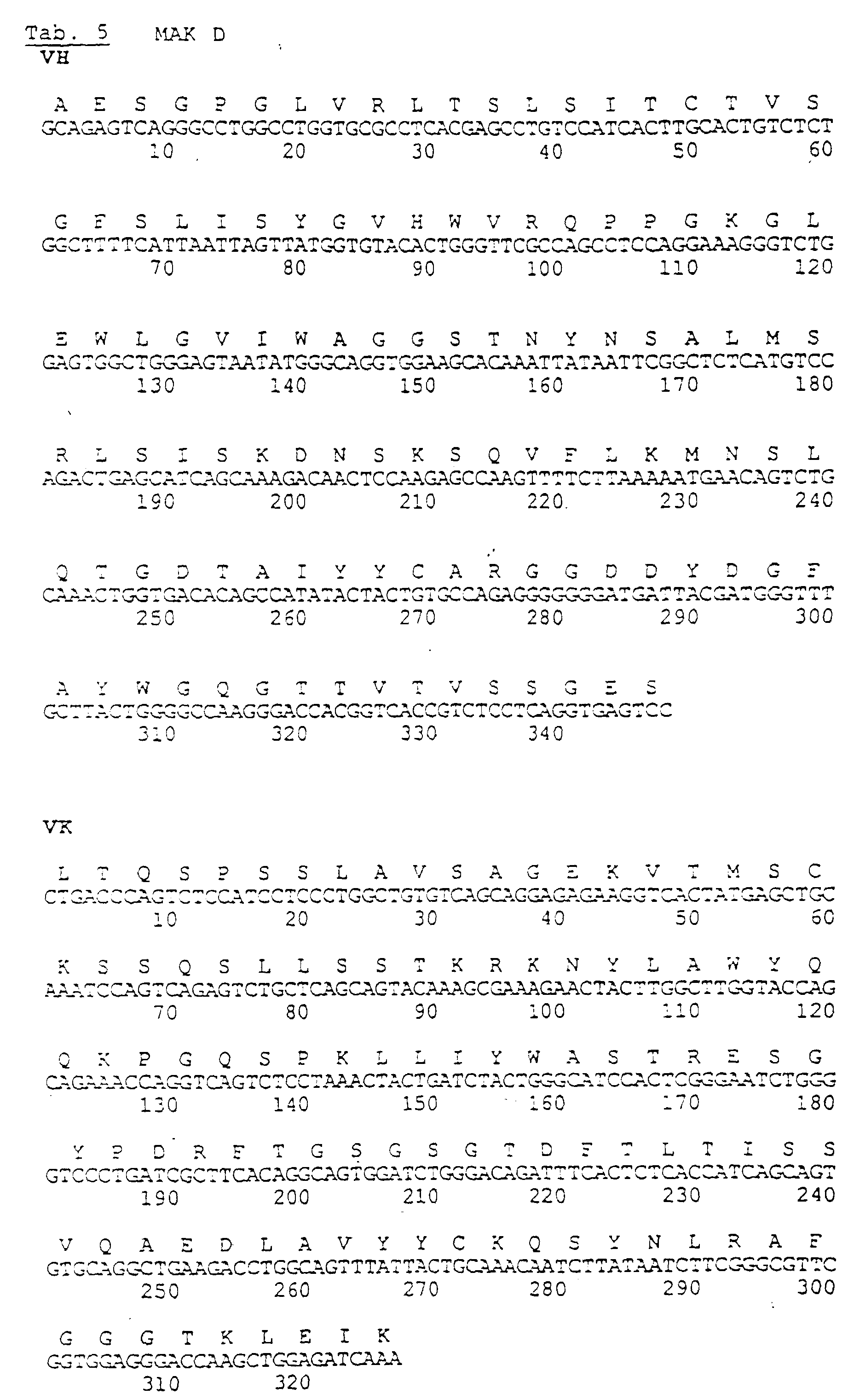

7. Rezeptoren nach Anspruch 1 bis Anspruch 6 als Arzneimittel.

8. Verfahren zur Herstellung von Rezeptoren nach Anspruch 1, 2, 3, 4, 5 oder 6, dadurch gekennzeichnet, daß die für die schweren Ketten-Antikörperteile kodierenden DNA-Fragmente mittels Linker verbunden und in einem Expressionssystem zusammen mit den Genen für die leichten Ketten exprimiert werden.

9. Bispezifische, tetravalente Rezeptoren nach Formel I, bei denen die C_{H}3. Domänen durch humane C_{H}1 Domänen ersetzt sind.

## Patentansprüche (Patentansprüche für folgende(n) Vertragsstaat(en): GR, ES)

1. Verfahren zur Herstellung von bispezifischen, tetravalenten Rezeptoren nach Formel I 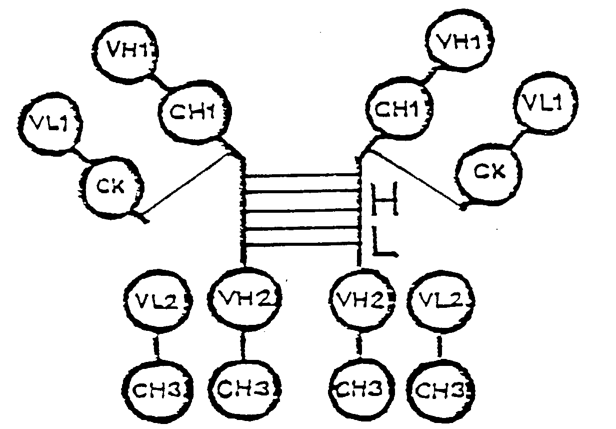 oder Formel II 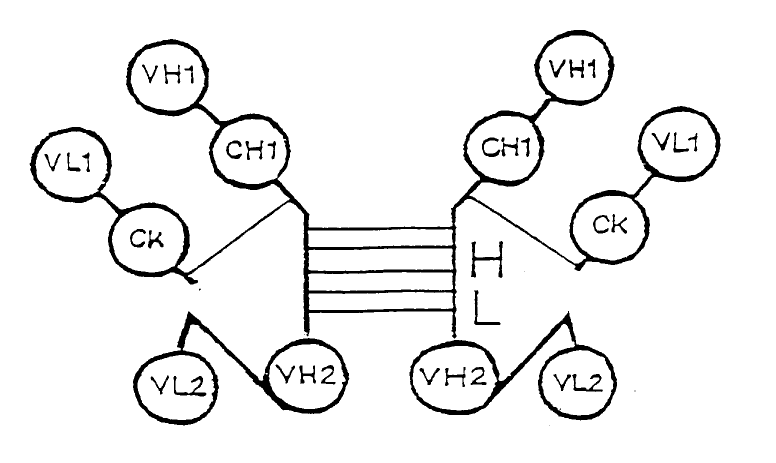 dadurch gekennzeichnet, daß nur die zusammengehörigen V_{L} und V_{H}-Paarungen gebildet werden und die C_{H}1 Domänen mit den V_{H}2 Domänen über 1 bis 10 Hinge-Regionen H und einen geeigneten Peptidlinker L verbunden werden, wobei die Disulfidbrücken (dünne Striche) im Bereich der Hinge-Regionen die Dimerisierung der bivalenten Halbmoleküle bewirken.

2. Verfahren nach Anspruch 1, dadurch gekennzeichnet, daß eine Spezifität gegen animale oder humane tumorassoziierte Antigene gerichtet ist.

3. Verfahren nach Anspruch 1, dadurch gekennzeichnet, daß eine Spezifität katalytische oder enzymatische Aktivität besitzt.

4. Verfahren nach Anspruch 1, dadurch gekennzeichnet, daß eine Spezifität gegen animale oder humane tumorassoziierte Antigene und die andere Spezifität katalytische oder enzymatische Aktivität besitzt.

5. Verfahren nach Anspruch 1, dadurch gekennzeichnet, daß eine Spezifität gegen animale oder humane tumorassoziierte Antigene gerichtet ist und eine andere Spezifität gegen ein Komplexon gerichtet ist.

6. Verfahren nach Anspruch 1, 2, 4 oder 5, dadurch gekennzeichnet, daß eine Spezifität von den monoklonalen Antikörpern mit den variablen Regionen gemäß Tab. 2, 3, 4 oder 5 mit folgenden Sequenzen stammt:

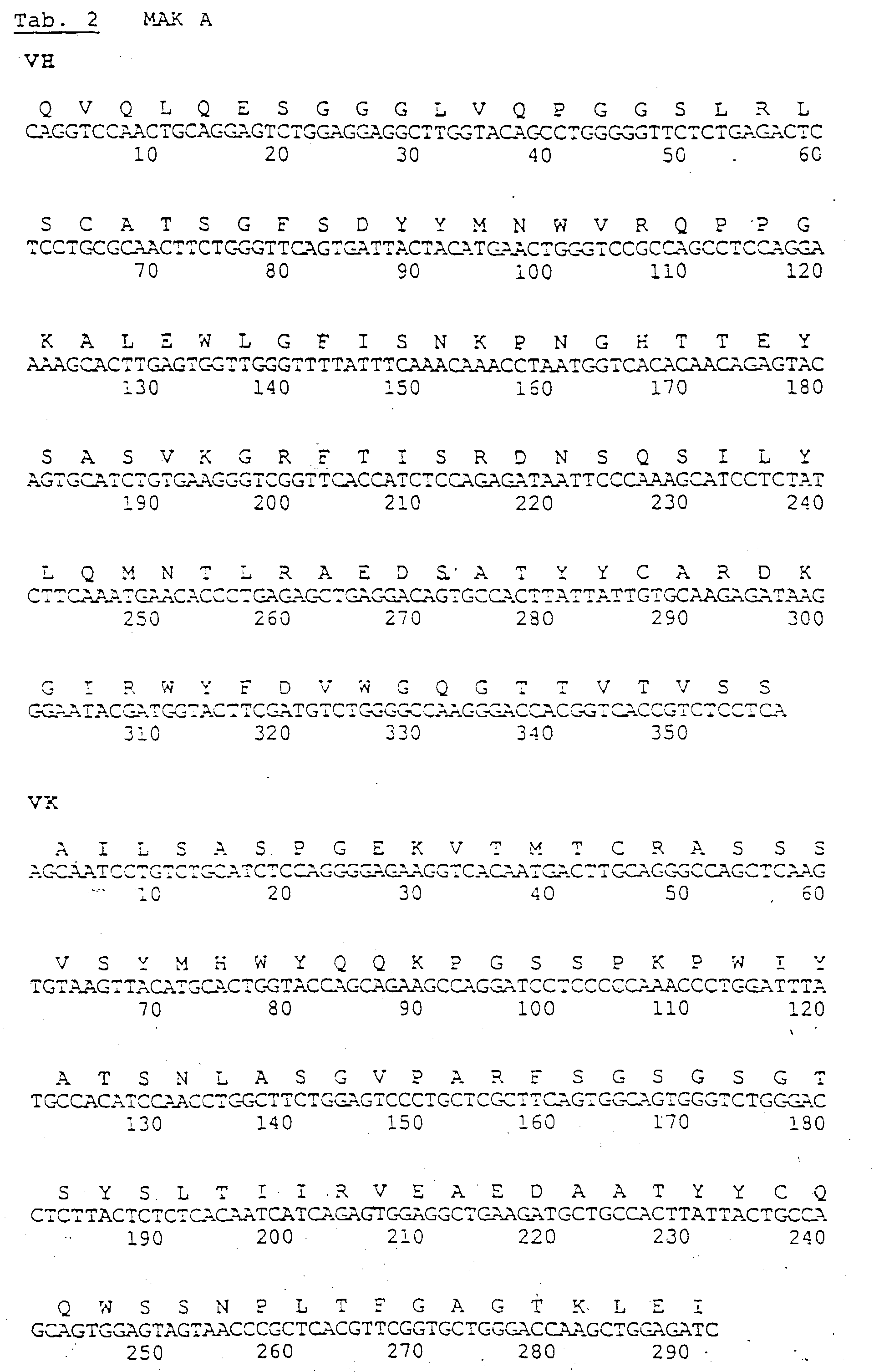

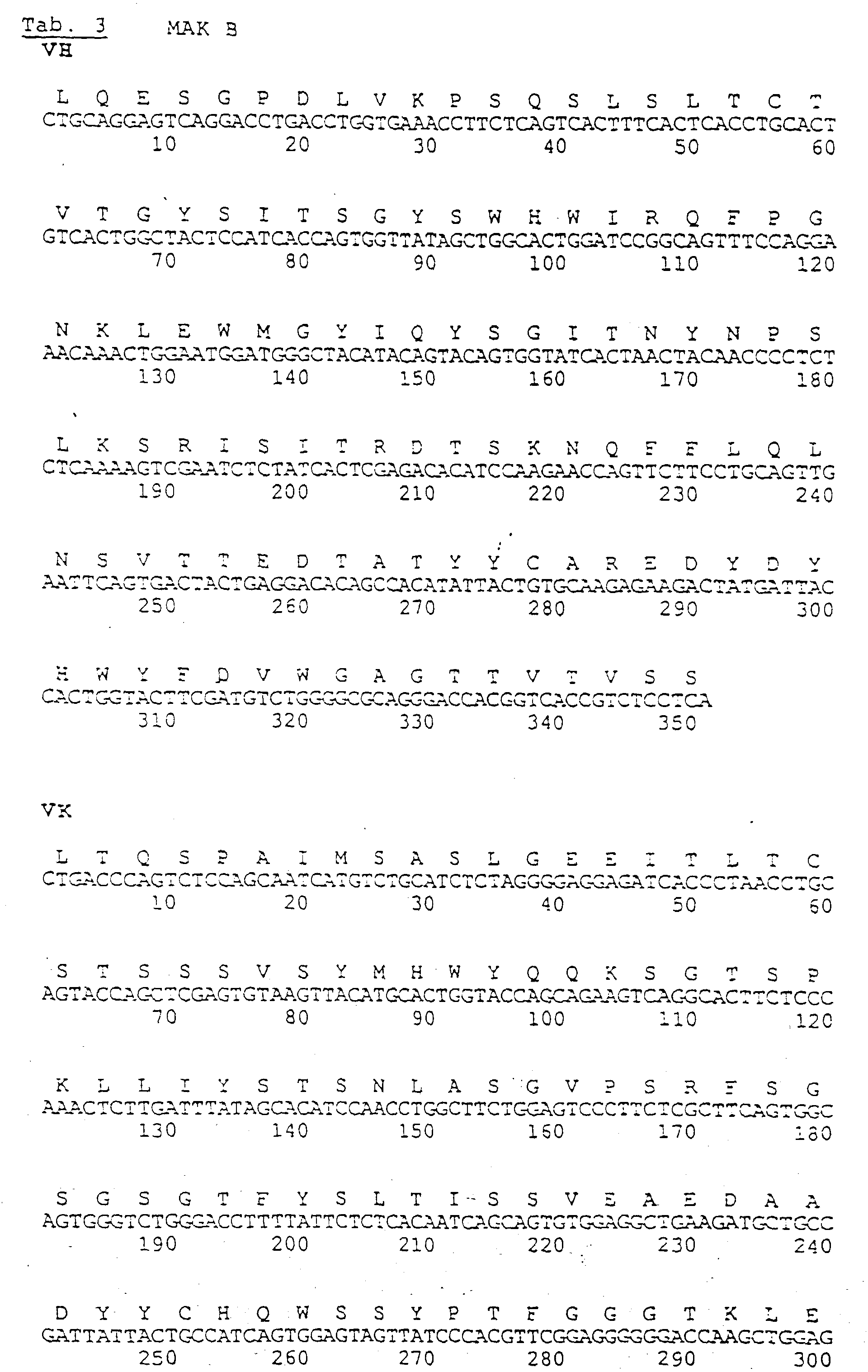

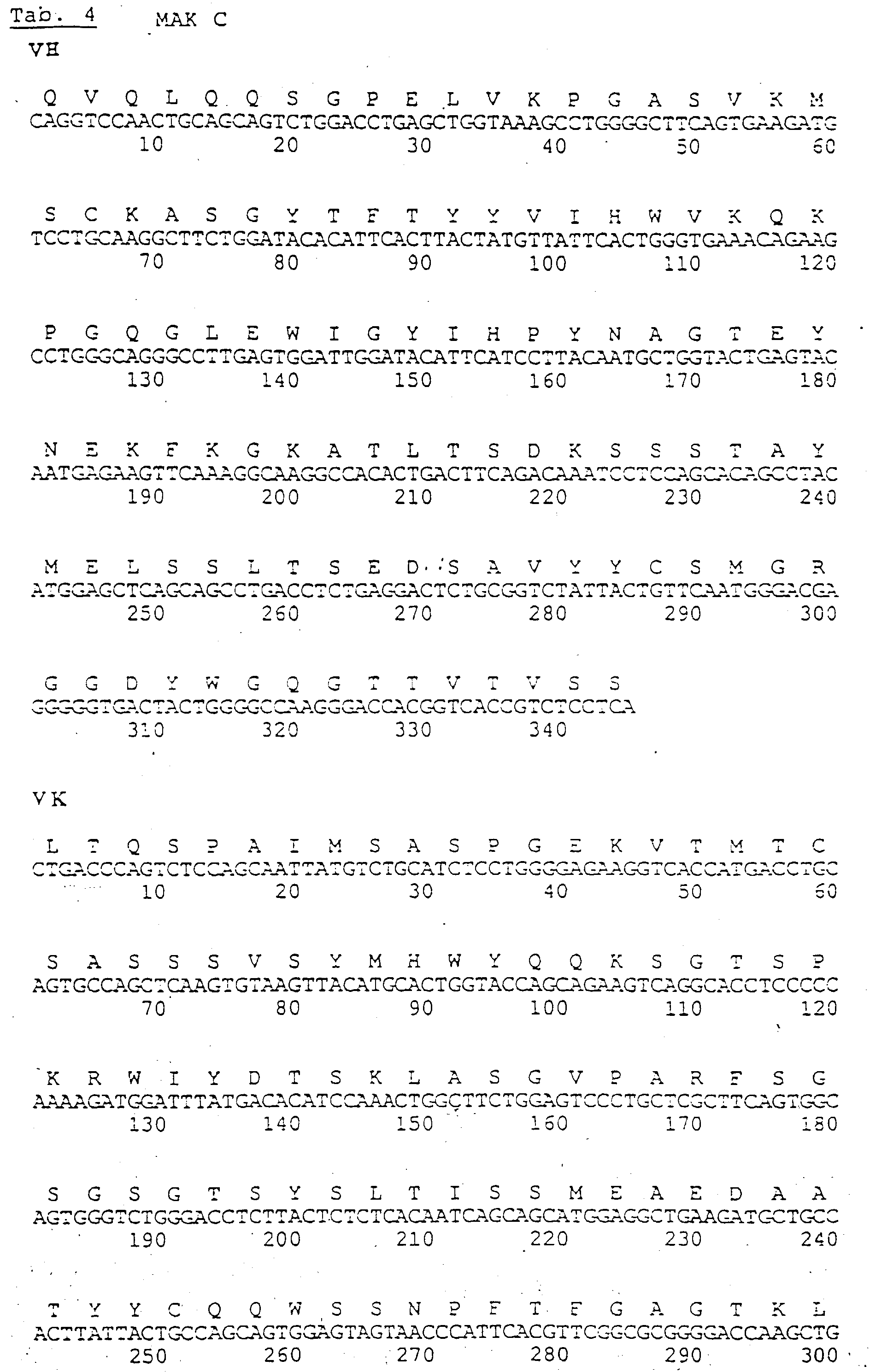

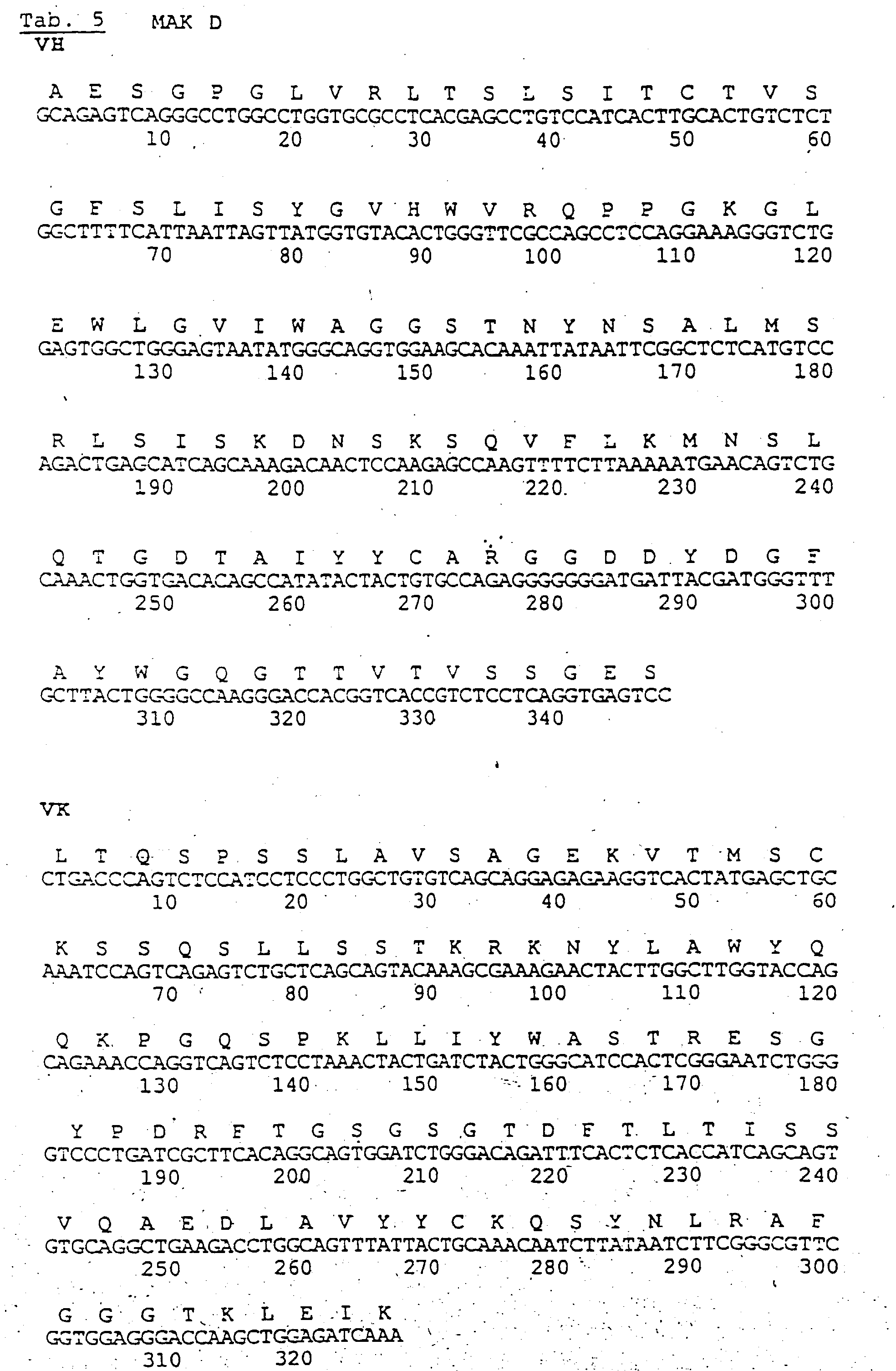

7. Verfahren nach Anspruch 1 bis Anspruch 6 zur Verwendung als Arzneimittel.

8. Verfahren zur Herstellung von Rezeptoren nach Anspruch 1, 2, 3, 4, 5 oder 6, dadurch gekennzeichnet, daß die für die schweren Ketten-Antikörperteile kodierenden DNA-Fragmente mittels Linker verbunden und in einem Expressionssystem zusammen mit den Genen für die leichten Ketten exprimiert werden.

9. Verfahren zur Herstellung von bispezifischen, tetravalenten Rezeptoren nach Formel 1, bei denen die C_{H}3 Domänen durch humane C_{H}1 Domänen ersetzt werden.

## Claims (Claims for the following Contracting State(s): AT, BE, CH, DE, DK, FR, GB, IT, LI, NL, LU, PT, SE)

1. A bispecific tetravalent receptor of the formula I 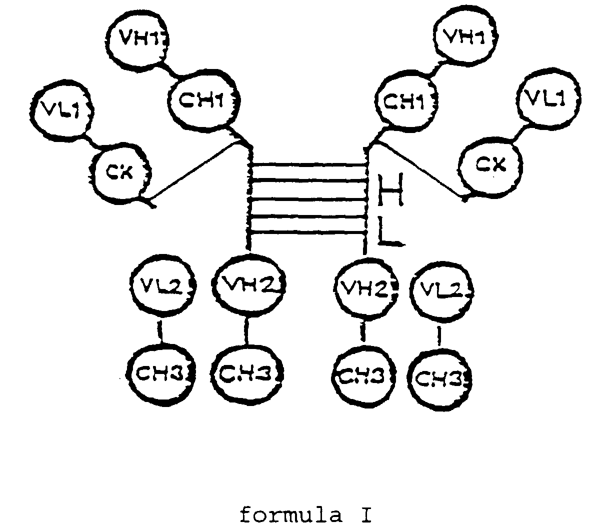 or formula II 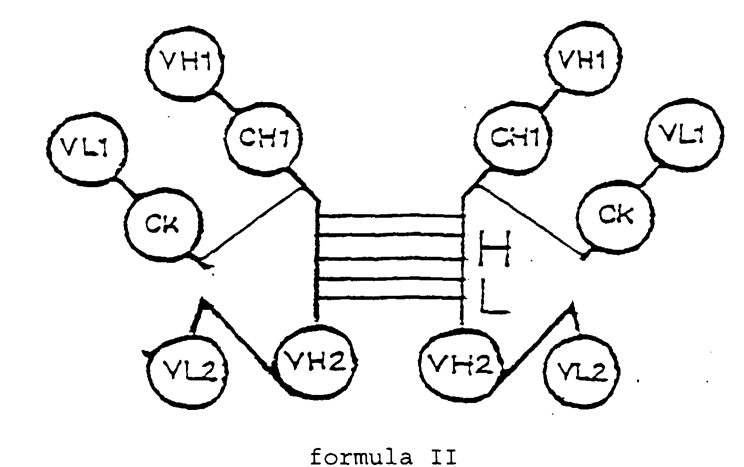 wherein only the V_{L} and V_{H} pairings which belong together are formed and the C_{H}1 domains are linked to the V_{H}2 domains via 1 to 10 hinge regions H and a suitable peptide linker L, where the disulfide bridges (thin lines) bring about the dimerization of the bivalent halves of the molecule in the region of the hinge regions.

2. A receptor as claimed in claim 1, wherein one specificity is directed against animal or human tumor-associated antigens.

3. A receptor as claimed in claim 1, wherein one specificity has catalytic or enzymatic activity.

4. A receptor as claimed in claim 1, wherein one specificity is directed against animal or human tumor-associated antigens and the other specificity has catalytic or enzymatic activity.

5. A receptor as claimed in claim 1, wherein one specificity is directed against animal or human tumor-associated antigens and another specificity is directed against a complexon.

6. A receptor as claimed in claim 1, 2, 4 or 5, wherein one specificity derives from the monoclonal antibodies with the variable regions shown in Tab. 2, 3, 4 or 5 having the following sequences:

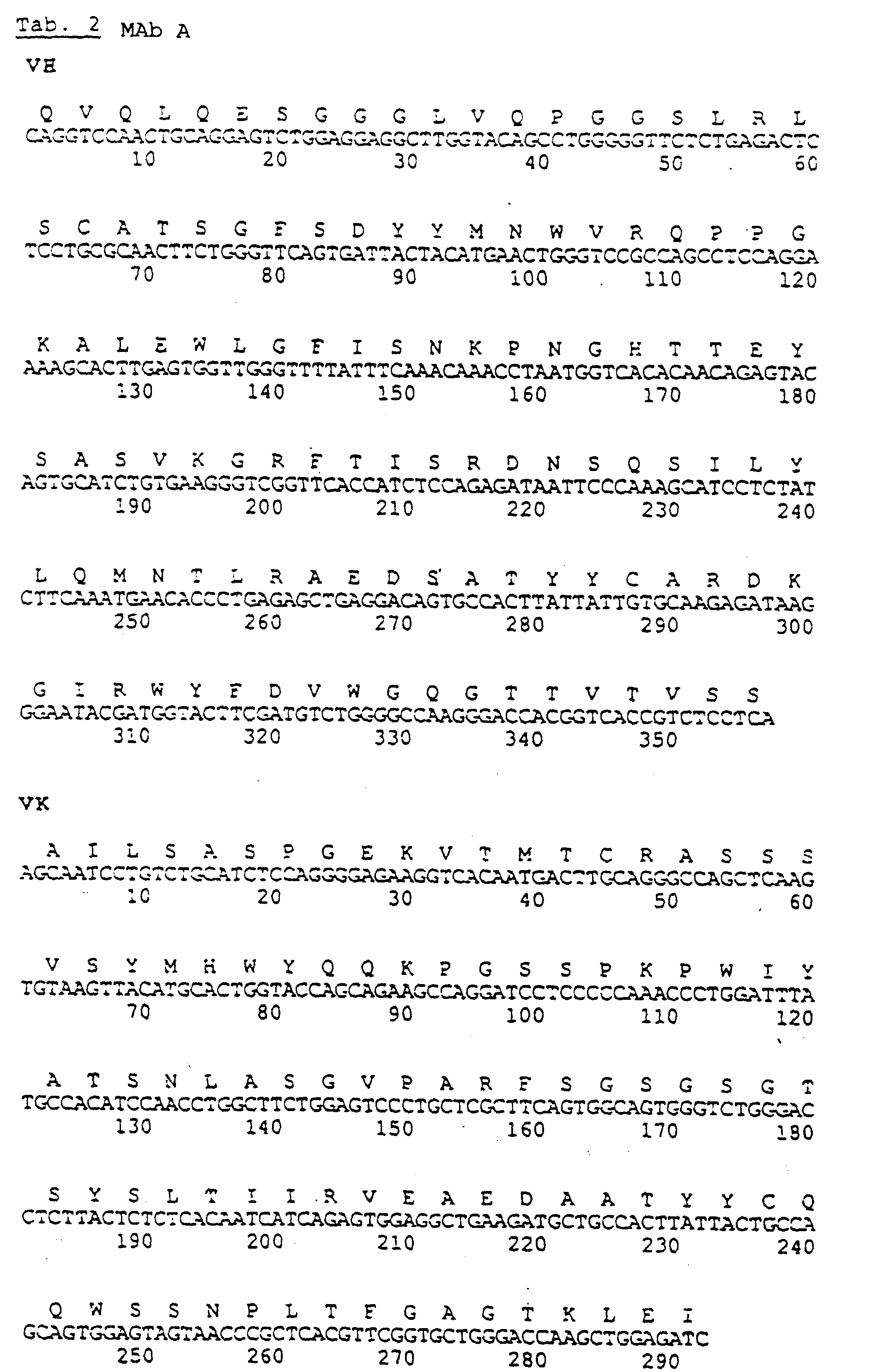

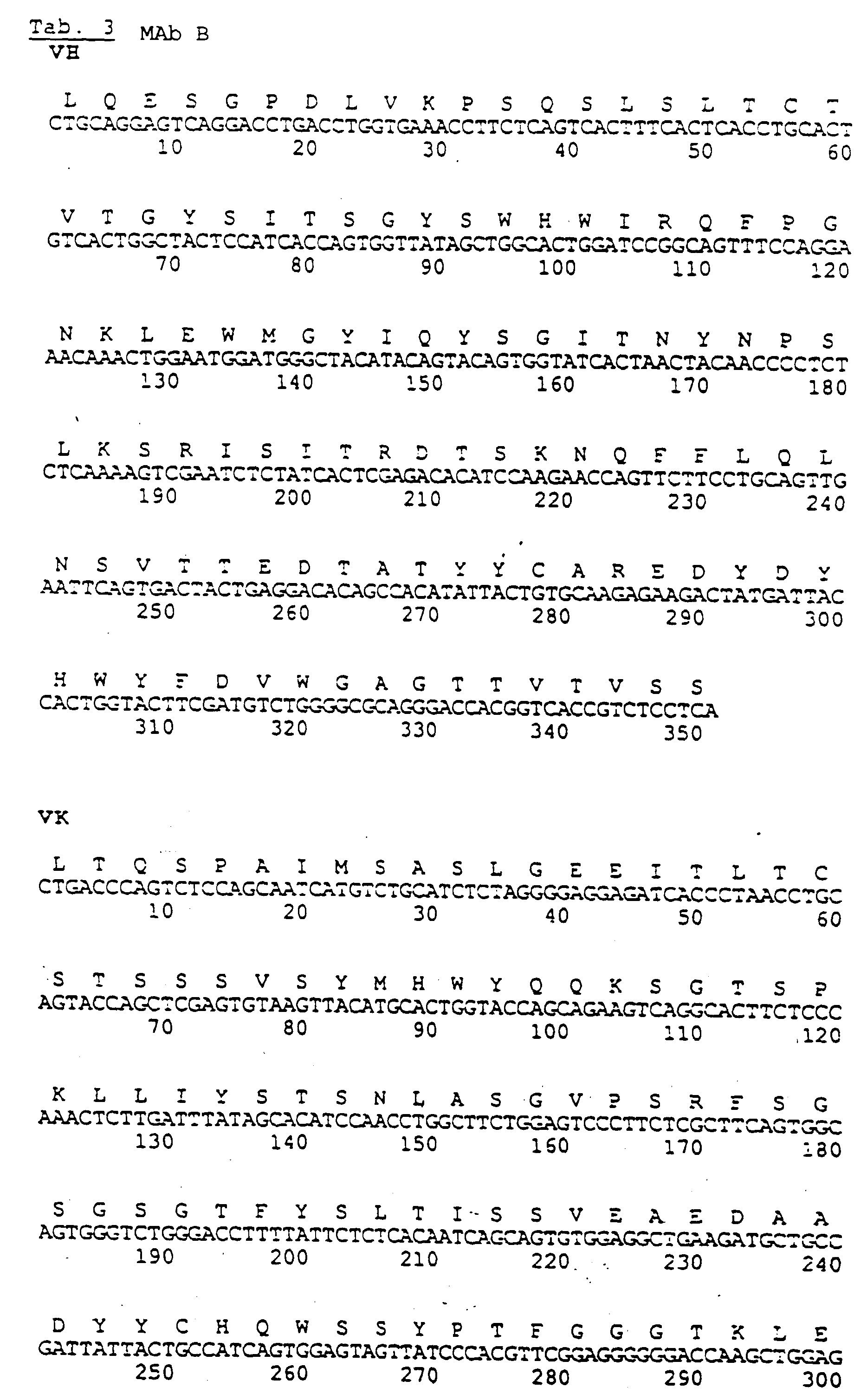

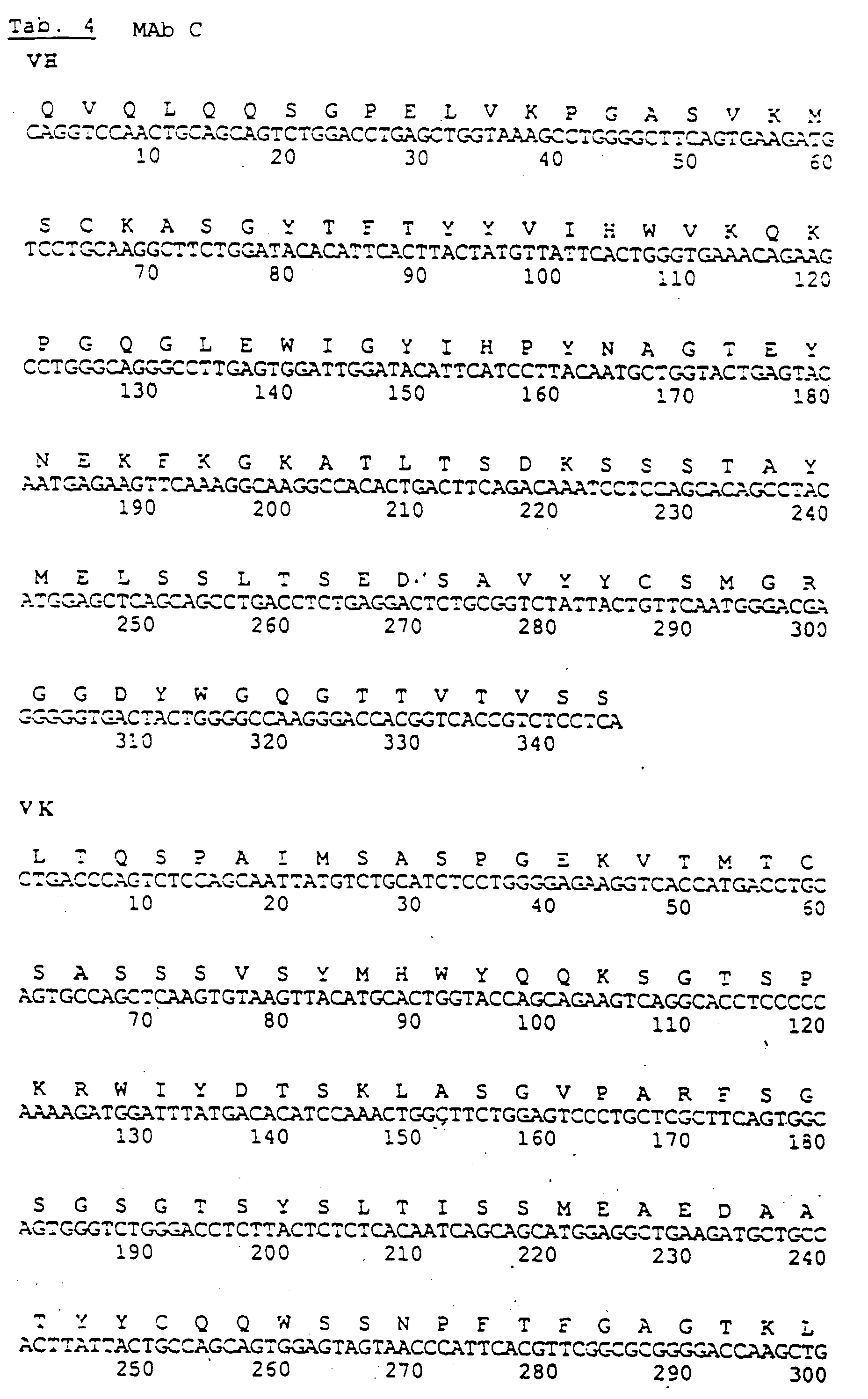

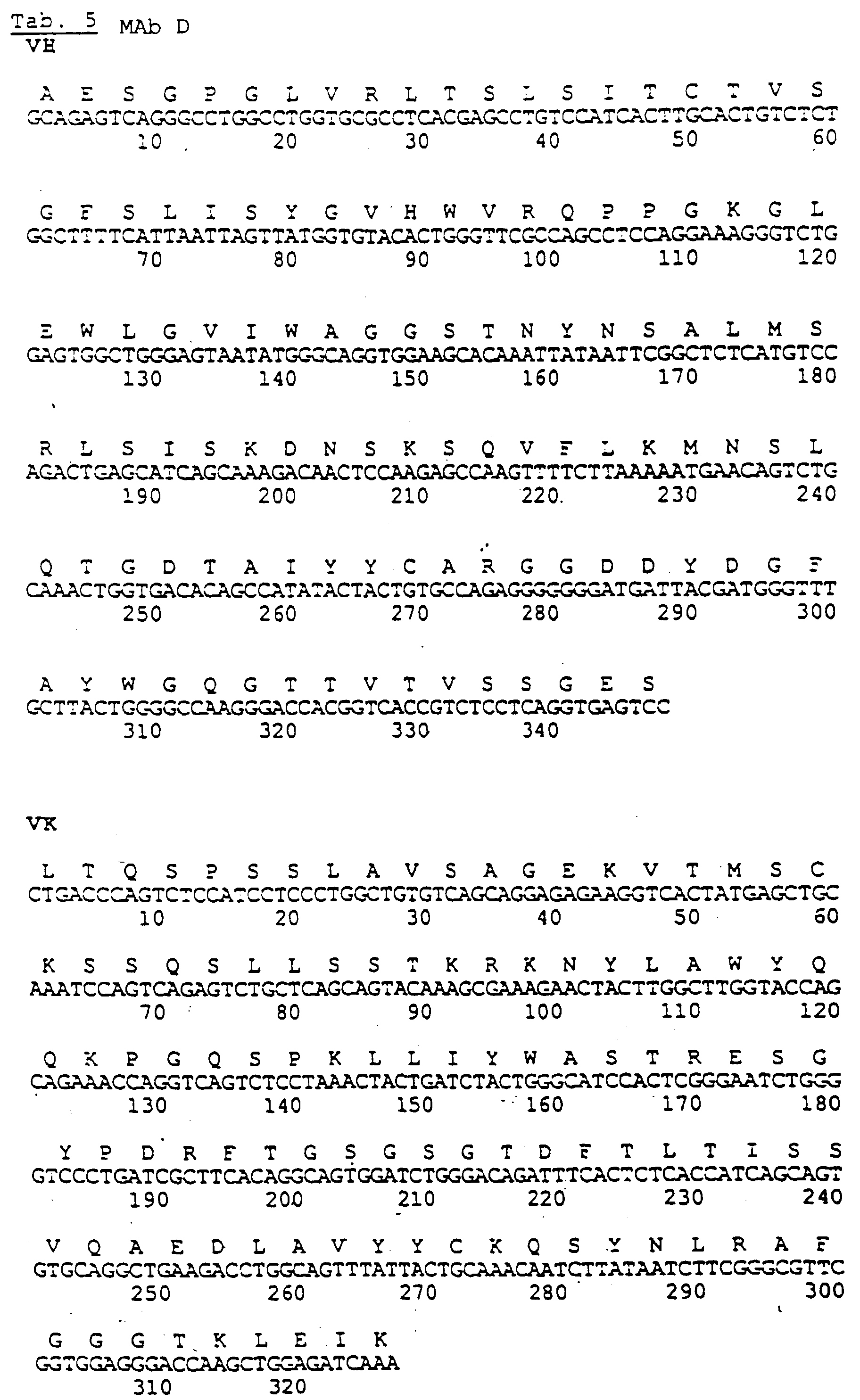

7. A receptor as claimed in claim 1 to claim 6 as pharmaceutical.

8. A process for the preparation of receptors as claimed in claim 1, 2, 3, 4, 5 or 6, which comprises the DNA fragments coding for the heavy chain antibody portions being connected by linkers and expressed in an expression system together with the genes for the light chains.

9. A bispecific tetravalent receptor of the formula I in which the C_{H}3 domains are replaced by human C_{H}1 domains.

## Claims (Claims for the following Contracting State(s): GR, ES)

1. A process for the preparation of a bispecific tetravalent receptor of the formula I 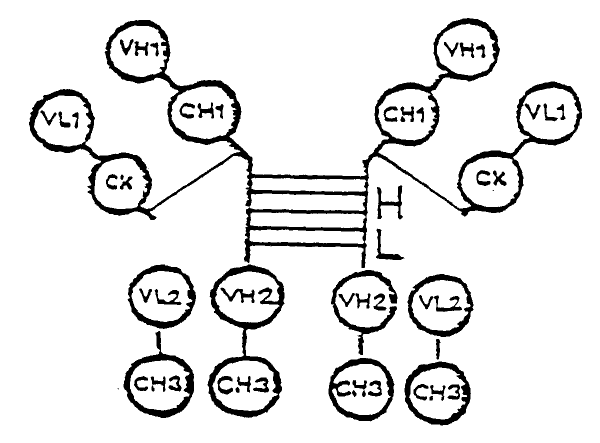 or formula II 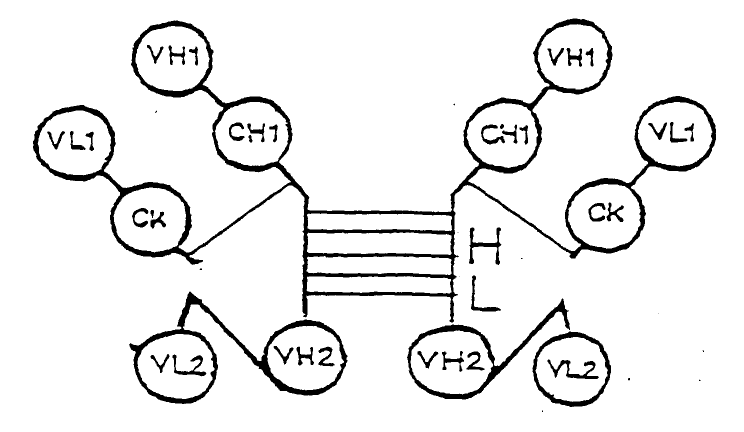 which comprises only the V_{L} and V_{H} pairings which belong together being formed and the C_{H}1 domains being linked to the V_{H}2 domains via 1 to 10 hinge regions H and a suitable peptide linker L, where the disulfide bridges (thin lines) bring about the dimerization of the bivalent halves of the molecule in the region of the hinge regions.

2. The process as claimed in claim 1, wherein one specificity is directed against animal or human tumor-associated antigens.

3. The process as claimed in claim 1, wherein one specificity has catalytic or enzymatic activity.

4. The process as claimed in claim 1, wherein one specificity is directed against animal or human tumor-associated antigens and the other specificity has catalytic or enzymatic activity.

5. The process as claimed in claim 1, wherein one specificity is directed against animal or human tumor-associated antigens and another specificity is directed against a complexon.

6. The process as claimed in claim 1, 2, 4 or 5, wherein one specificity derives from the monoclonal antibodies with the variable regions shown in Tab. 2, 3, 4 or 5 having the following sequences:

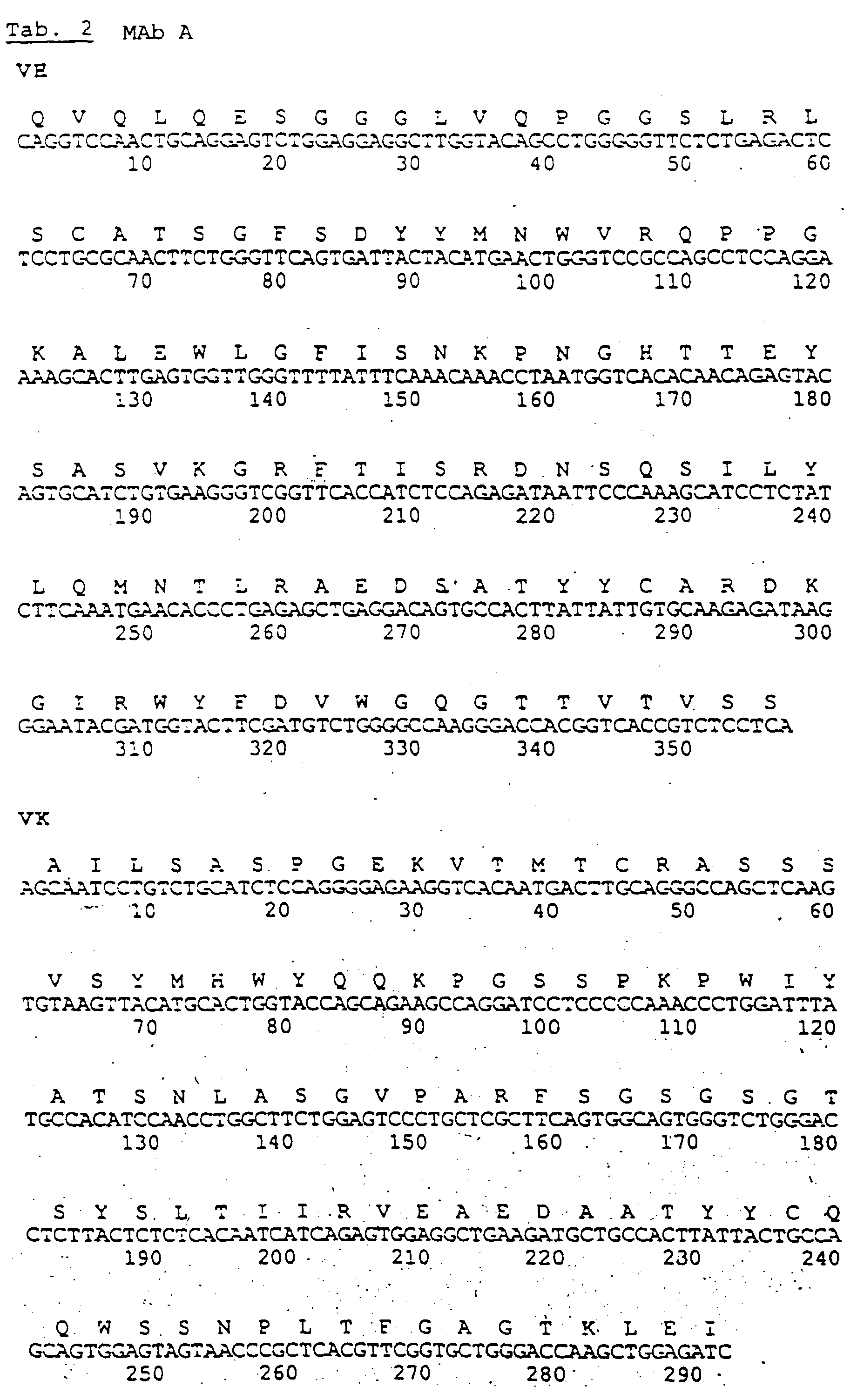

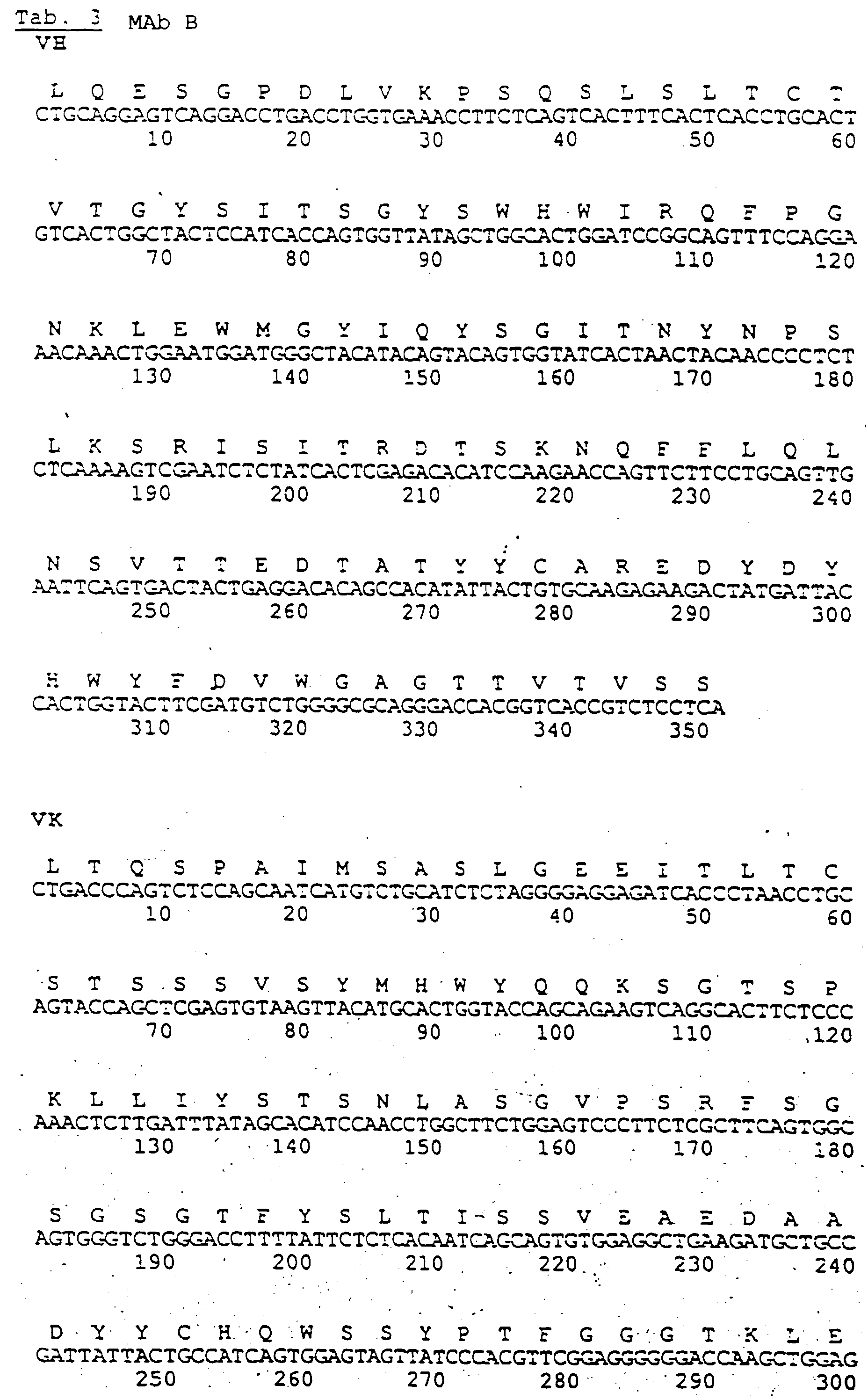

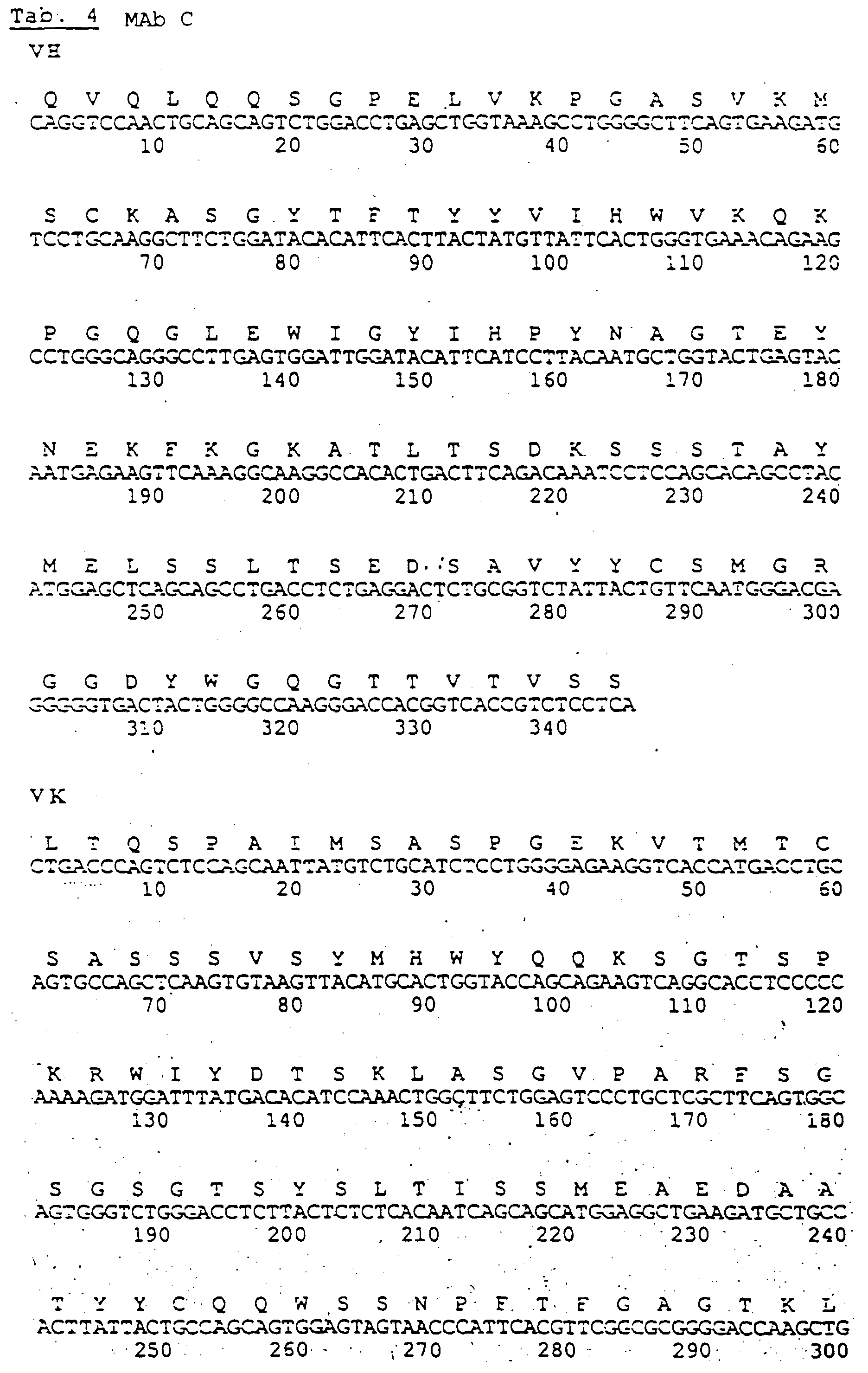

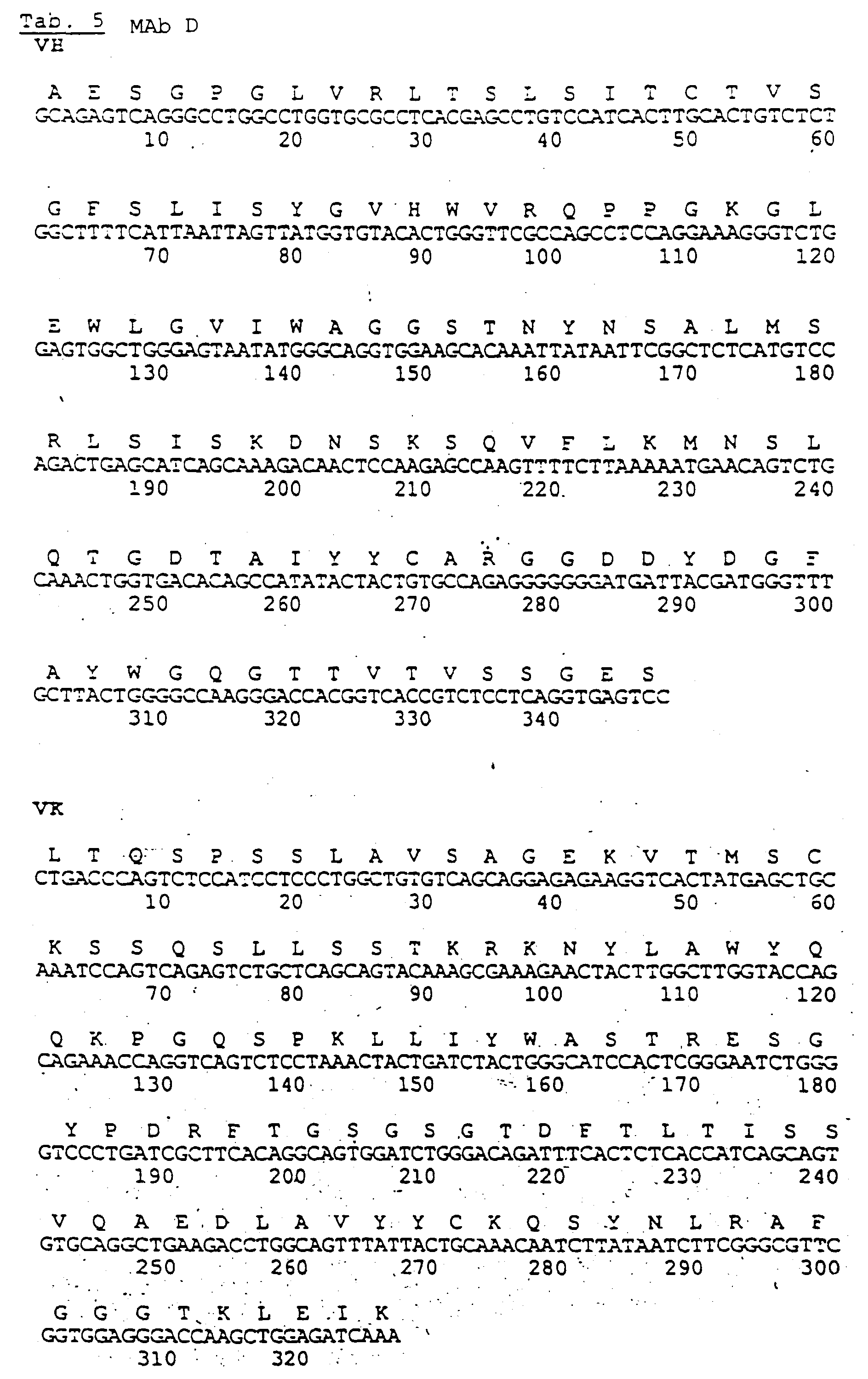

7. The process as claimed in claim 1 to claim 6 for use as pharmaceutical.

8. A process for the preparation of receptors as claimed in claim 1, 2, 3, 4, 5 or 6, which comprises the DNA fragments coding for the heavy chain antibody portions being connected by linkers and expressed in an expression system together with the genes for the light chains.

9. A process for the preparation of a bispecific tetravalent receptor of the formula I in which the C_{H}3 domains are replaced by human C_{H}1 domains.

## Revendications (Revendications pour l'(les) Etat(s) contractant(s) suivant(s): AT, BE, CH, DE, DK, FR, GB, IT, LI, NL, LU, PT, SE)

1. Récepteurs tétravalents bispécifiques de formule I 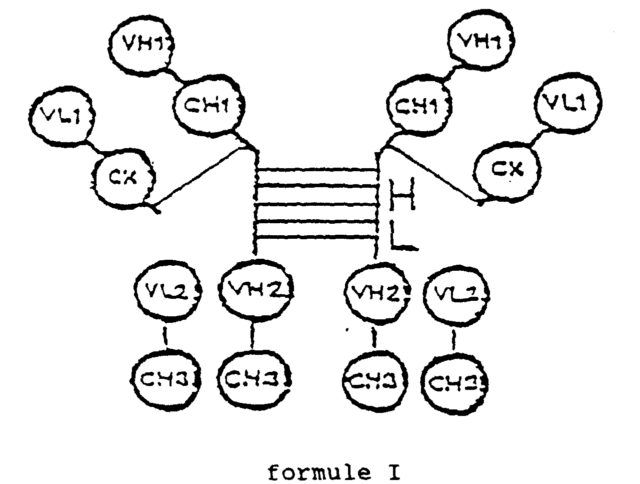 ou de formule II 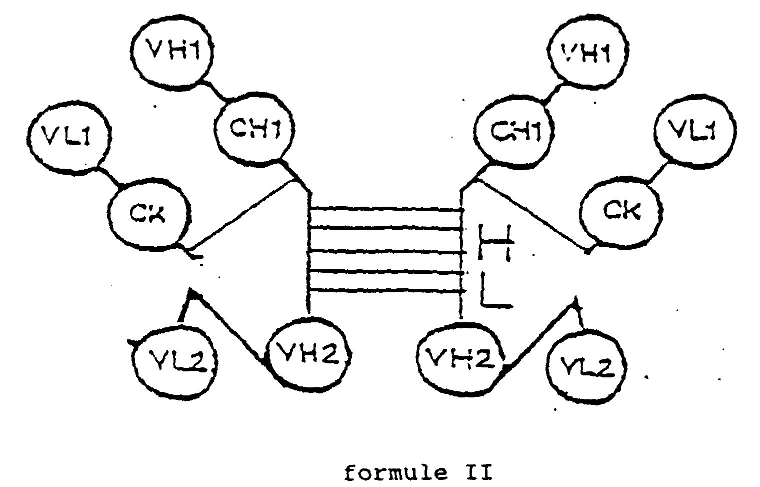 caractérisés en ce que seuls sont formés les appariements V_{L} et V_{H} correspondants et les domaines C_{H}1 sont reliés aux domaines V_{H}2 par l'intermédiaire de 1 à 10 régions charnières H et d'un lieur peptidique L approprié, les ponts disulfure (traits fins) provoquant la dimérisation des demi-molécules divalentes dans la zone des régions charnières.

2. Récepteurs selon la revendication 1, caractérisés en ce qu'une spécificité est dirigée contre des antigènes humains ou animaux associés à des tumeurs.

3. Récepteurs selon la revendication 1, caractérisés en ce qu'une spécificité a une activité catalytique ou enzymatique.

4. Récepteurs selon la revendication 1, caractérisés en ce qu'une spécificité est dirigée contre des antigènes humains ou animaux associés à des tumeurs, et l'autre spécificité a une activité catalytique ou enzymatique.

5. Récepteurs selon la revendication 1, caractérisés en ce qu'une spécificité est dirigée contre des antigènes humains ou animaux associés à des tumeurs, et une autre spécificité est dirigée contre un complexon.

6. Récepteurs selon la revendication 1, 2, 4 ou 5, caractérisés en ce qu'une spécificité dérive des anticorps monoclonaux comportant les régions variables selon le tableau 2, 3, 4 ou 5, ayant les séquences suivantes:

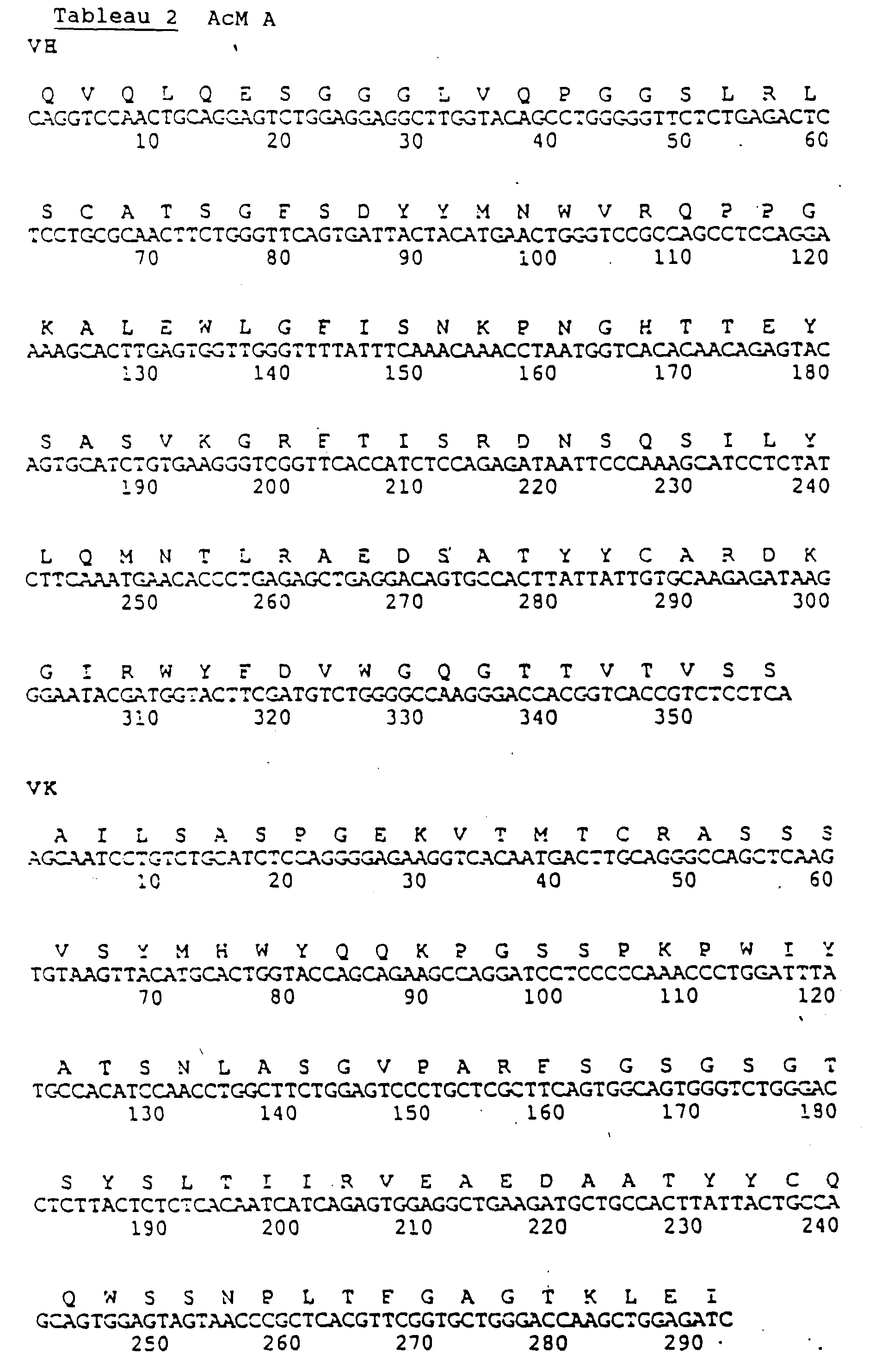

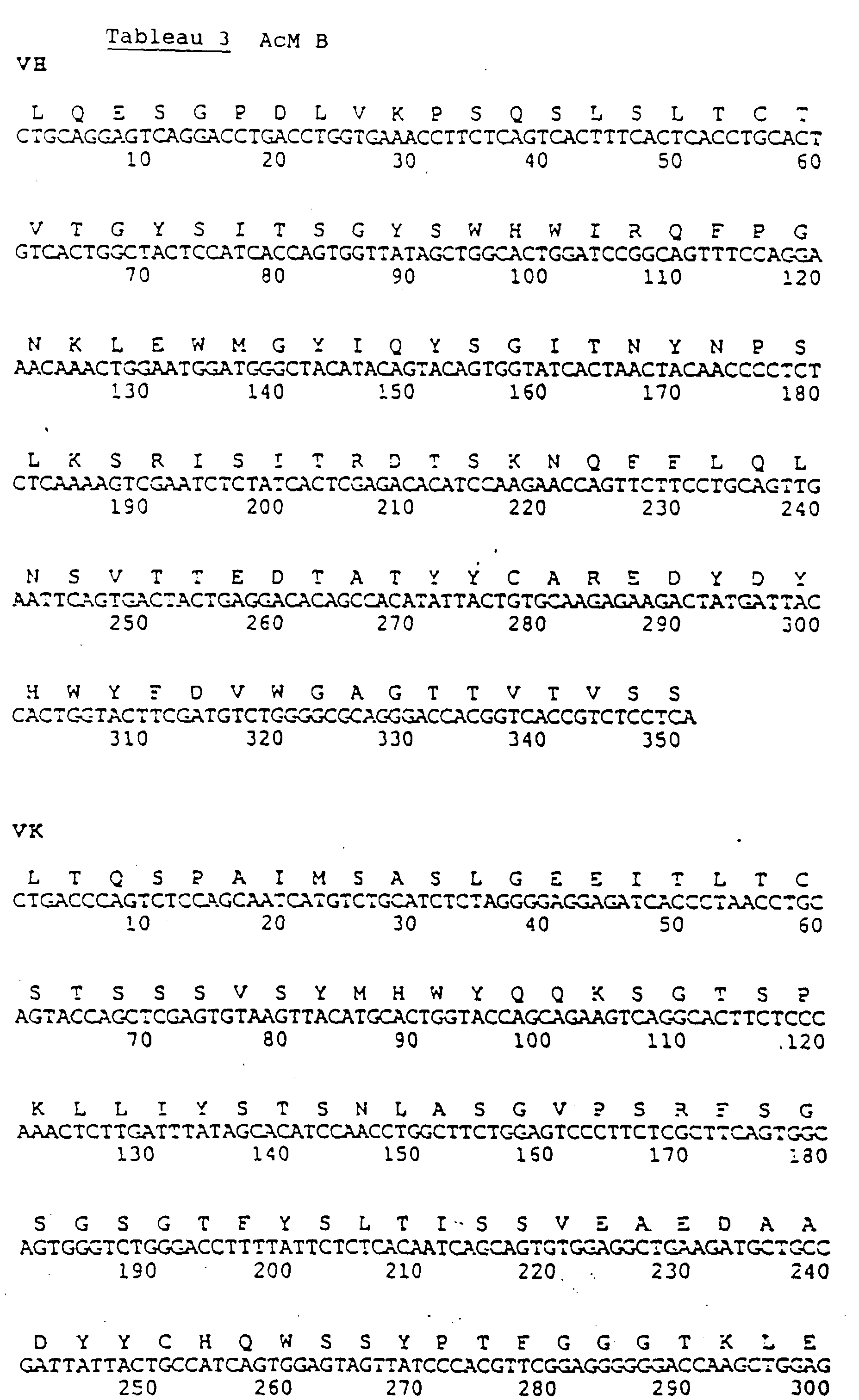

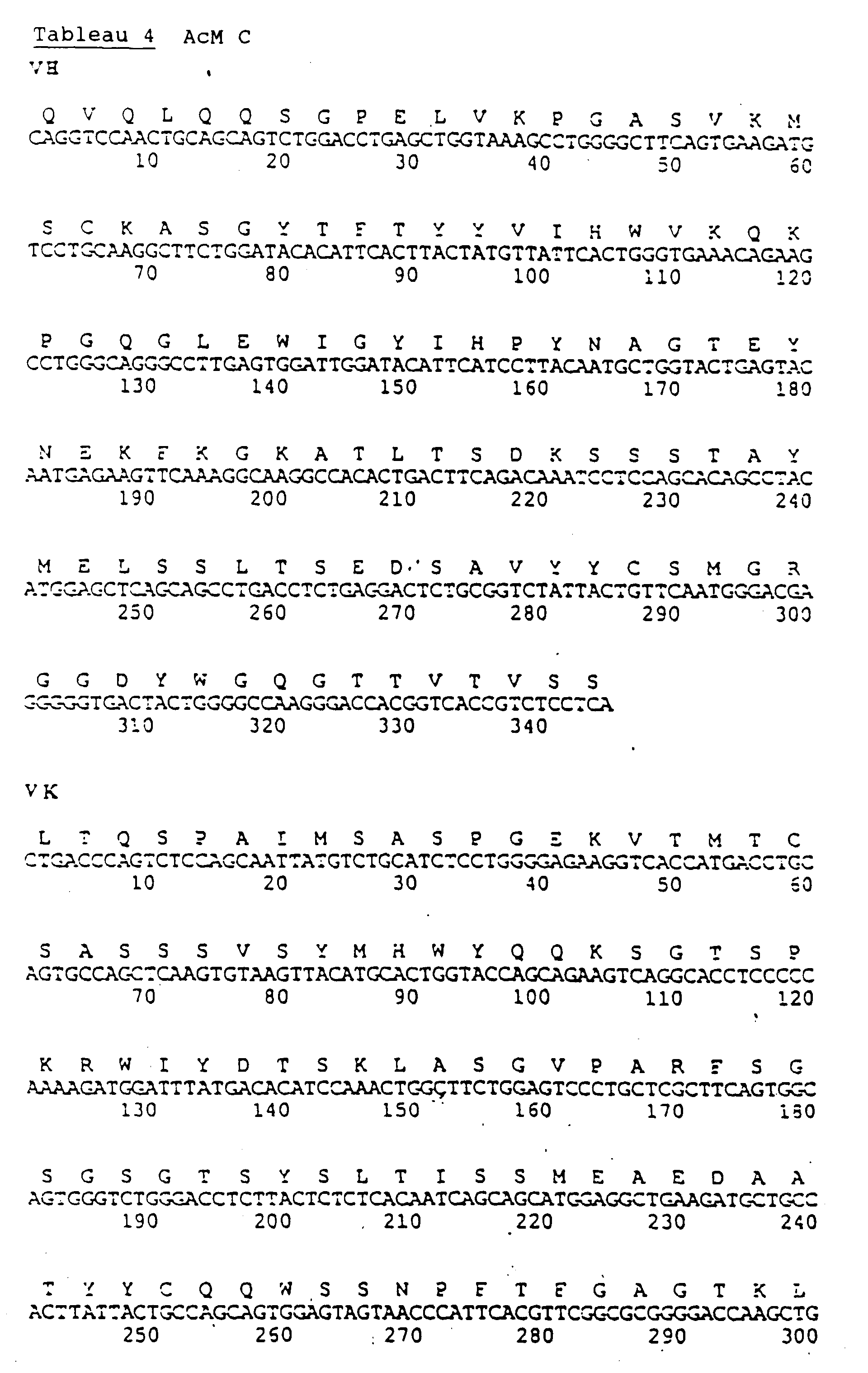

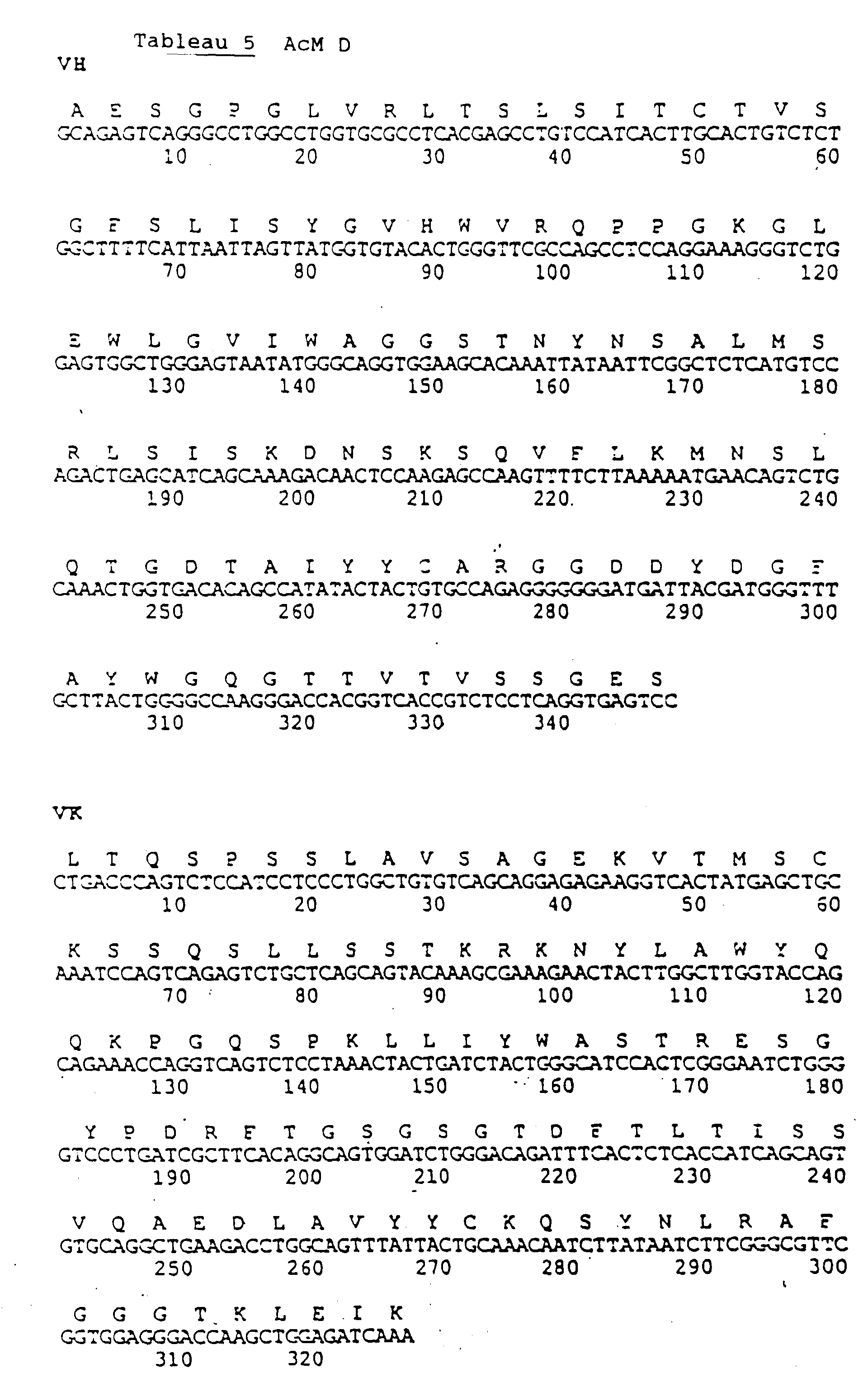

7. Récepteurs selon les revendications 1 à 6, en tant que médicaments.

8. Procédé pour la production de récepteurs selon la revendication 1, 2, 3, 4, 5 ou 6, caractérisé en ce que les fragments d'ADN codant pour les segments d'anticorps de type chaîne lourde sont assemblés au moyen de lieurs et exprimés dans un système d'expression, conjointement avec les gènes codant pour les chaînes légères.

9. Récepteurs tétravalents bispécifiques de formule I, dans lesquels les domaines C_{H}3 sont remplacés par des domaines C_{H}1 humains.

## Revendications (Revendications pour l'(les) Etat(s) contractant(s) suivant(s): GR, ES)

1. Procédé pour la production de récepteurs tétravalents bispécifiques de formule I 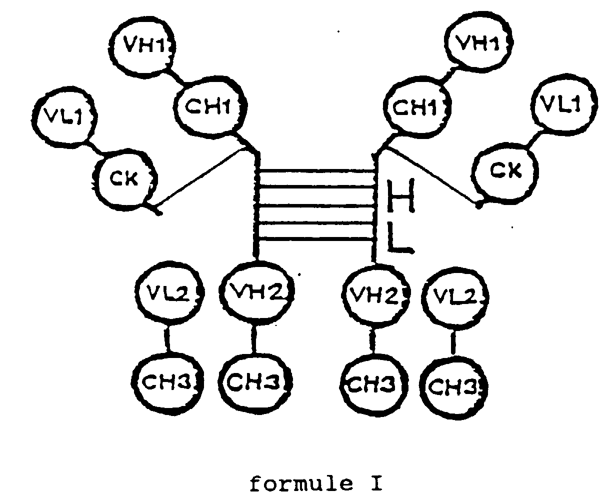 ou de formule II 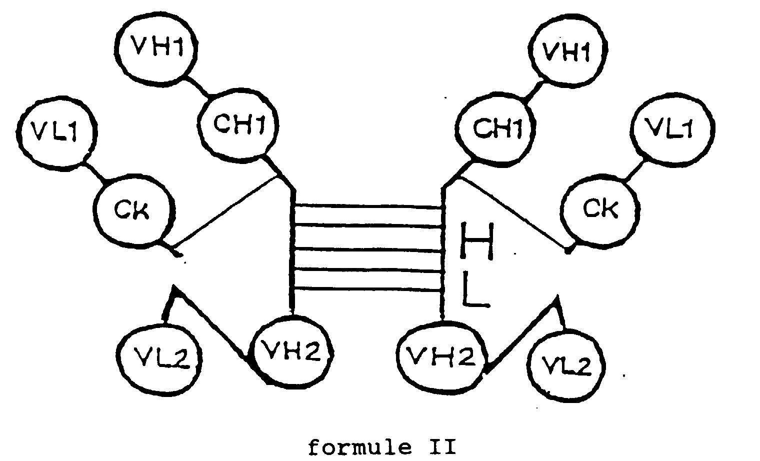 caractérisés en ce que seuls sont formés les appariements V_{L} et V_{H} correspondants et les domaines C_{H}1 sont reliés aux domaines V_{H}2 par l'intermédiaire de 1 à 10 régions charnières H et d'un lieur peptidique L approprié, les ponts disulfure (traits fins) provoquant la dimérisation des demi-molécules divalentes dans la zone des régions charnières.

2. Procédé selon la revendication 1, caractérisé en ce qu'une spécificité est dirigée contre des antigènes humains ou animaux associés à des tumeurs.

3. Procédé selon la revendication 1, caractérisé en ce qu'une spécificité a une activité catalytique ou enzymatique.

4. Procédé selon la revendication 1, caractérisé en ce qu'une spécificité est dirigée contre des antigènes humains ou animaux associés à des tumeurs, et l'autre spécificité a une activité catalytique ou enzymatique.

5. Procédé selon la revendication 1, caractérisé en ce qu'une spécificité est dirigée contre des antigènes humains ou animaux associés à des tumeurs, et une autre spécificité est dirigée contre un complexon.

6. Procédé selon la revendication 1, 2, 4 ou 5, caractérisé en ce qu'une spécificité dérive des anticorps monoclonaux comportant les régions variables selon le tableau 2, 3, 4 ou 5, ayant les séquences suivantes:

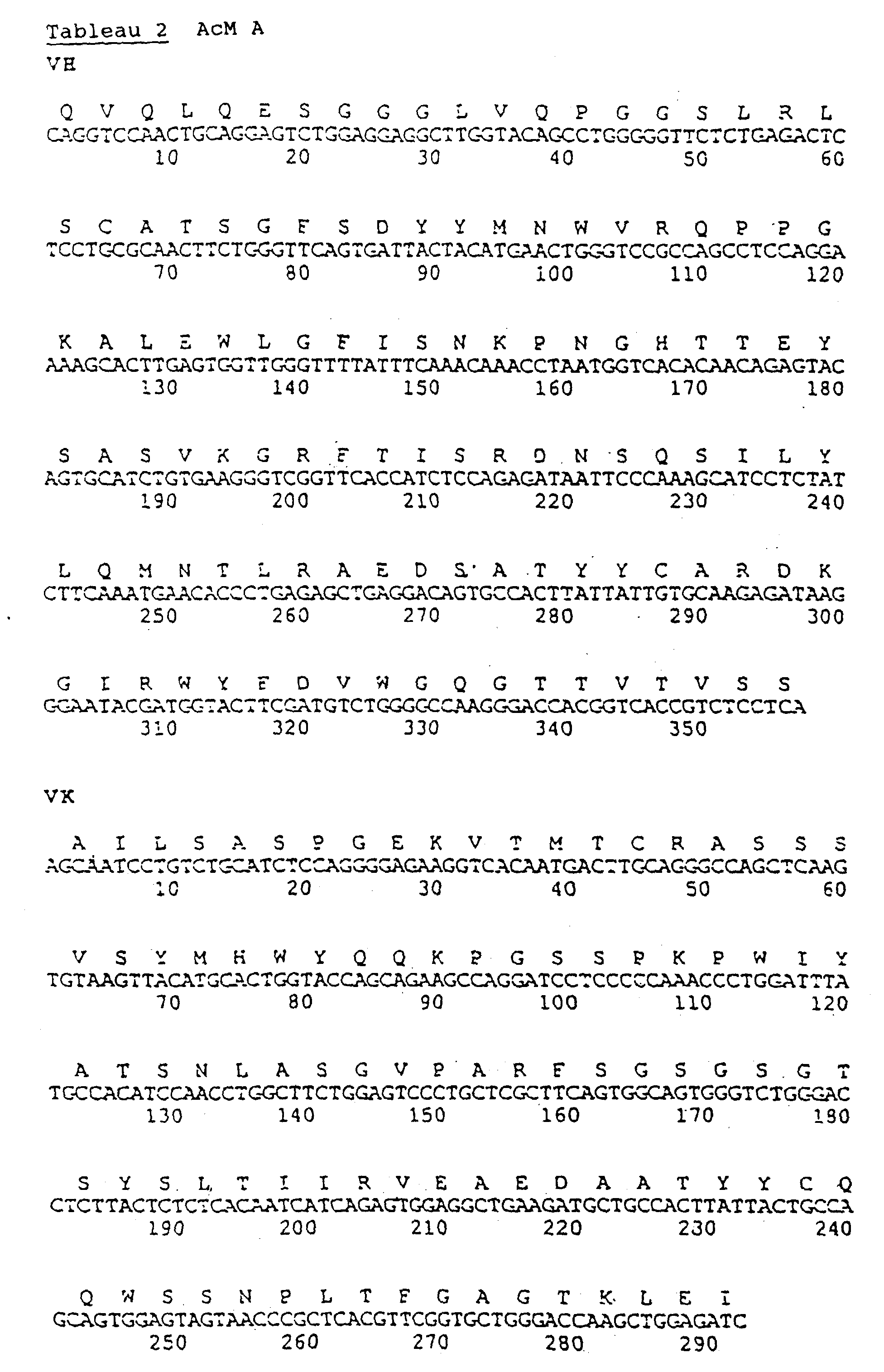

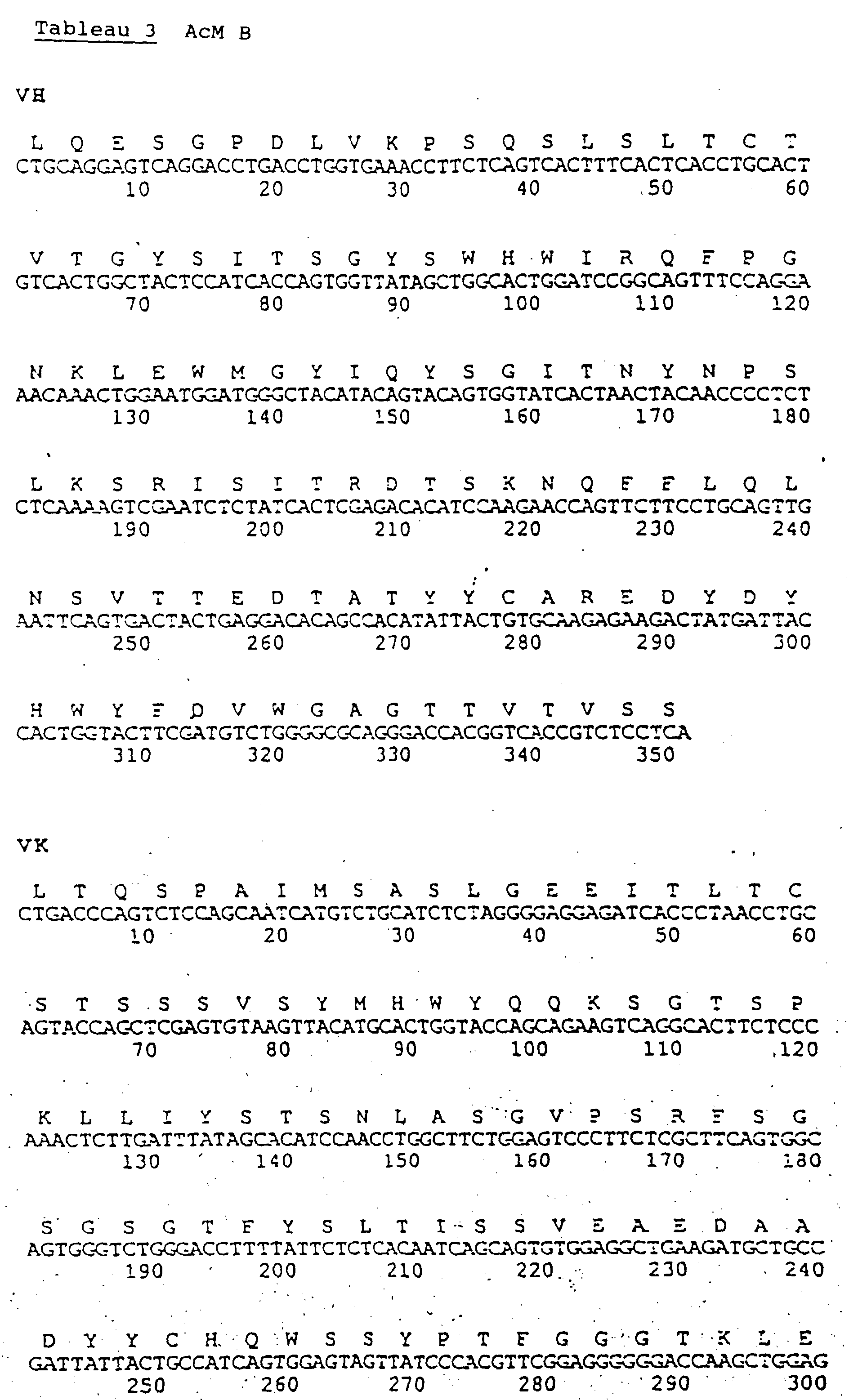

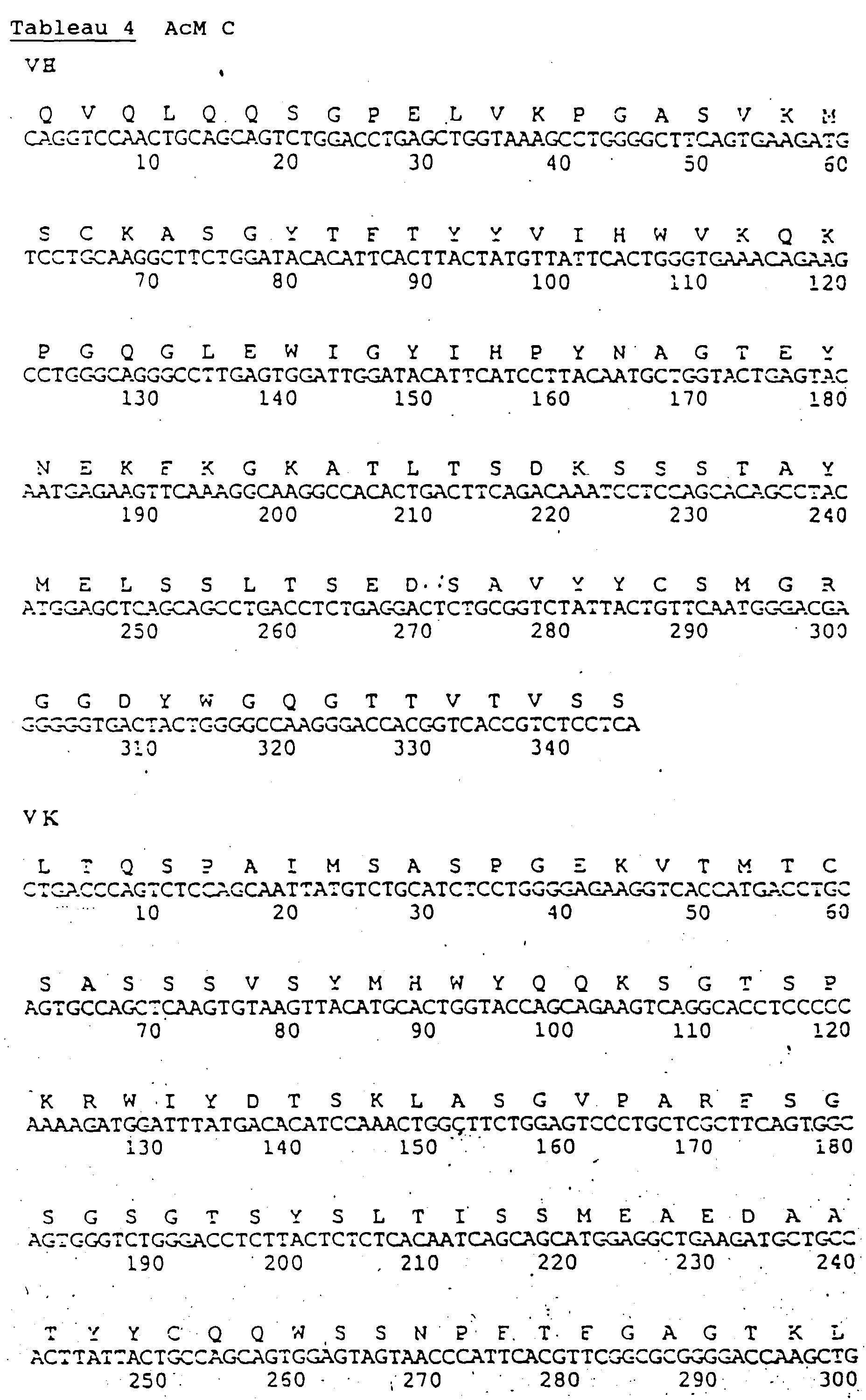

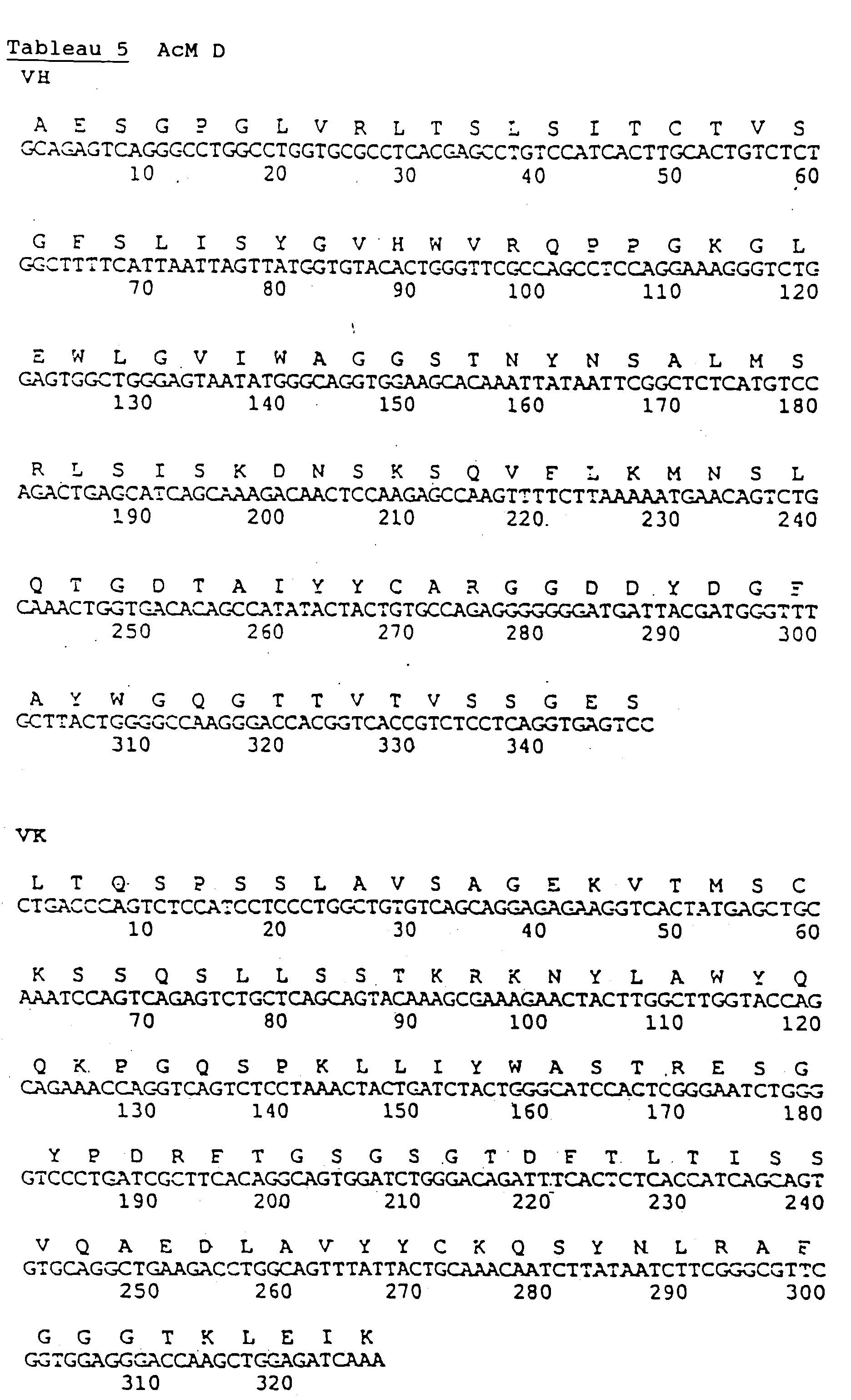

7. Procédé selon les revendications 1 à 6, pour utilisation des récepteurs en tant que médicaments.

8. Procédé pour la production de récepteurs selon la revendication 1, 2, 3, 4, 5 ou 6, caractérisé en ce que les fragments d'ADN codant pour les segments d'anticorps de type chaîne lourde sont assemblés au moyen de lieurs et exprimés dans un système d'expression, conjointement avec les gènes codant pour les chaînes légères.

9. Procédé pour la production de récepteurs tétravalents bispécifiques de formule I, dans lesquels les domaines C_{H}3 sont remplacés par des domaines C_{H}1 humains.
